(19) Europäisches Patentamt
European Patent Office
Office européen des brevets

(11) EP 0 816 334 B1

(12) **EUROPÄISCHE PATENTSCHRIFT**

(45) Veröffentlichungstag und Bekanntmachung des Hinweises auf die Patenterteilung:
**29.08.2001 Patentblatt 2001/35**

(51) Int Cl.$^7$: **C07C 403/14**, C07C 45/51, C07C 47/277, C07C 45/67, C07C 47/21

(21) Anmeldenummer: **97110253.8**

(22) Anmeldetag: **23.06.1997**

# (54) Herstellung von Polyen(di)aldehyden

Preparation of polyenic (di)aldehydes

Préparation de (di)aldéhydes polyéniques

(84) Benannte Vertragsstaaten:
**AT BE CH DE DK ES FR GB IT LI NL**

(30) Priorität: **28.06.1996 EP 96110463**

(43) Veröffentlichungstag der Anmeldung:
**07.01.1998 Patentblatt 1998/02**

(73) Patentinhaber: **F. HOFFMANN-LA ROCHE AG
4070 Basel (CH)**

(72) Erfinder: **Rüttimann, August
4144 Arlesheim (CH)**

(74) Vertreter: **Kellenberger, Marcus, Dr. et al
F.Hoffmann-La Roche AG
Patent Department (PLP),
124 Grenzacherstrasse
4070 Basel (CH)**

(56) Entgegenhaltungen:
**FR-A- 2 338 241**

- **CHEMICAL ABSTRACTS, vol. 57, no. 13, 24.Dezember 1962 Columbus, Ohio, US; abstract no. 16671c, ZH.A. KRASNAYA ET AL. : "A new path for the synthesis of vitamin A from beta-ionylideneacetaldehyde" Seite 16671; Spalte 1; XP002041881 & ZH. OBSHCH. KHIM., Bd. 32, 1962, Seiten 64-70,**
- **CHEMICAL ABSTRACTS, vol. 52, no. 14, 25.Juli 1958 Columbus, Ohio, US; abstract no. 11737e, I.N. NAZAROV ET AL.: "Condensation of acetals with ethoxyisoprene. New method of synthesis of polyene aldehydes of isoprenoid type" Seite 11737; Spalte 1; XP002041882 & DOKLADY AKAD. NAUK S.S.S.R., Bd. 118, 1958, Seiten 716-719,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 816 334 B1

## Beschreibung

Die vorliegende Erfindung betrifft ein neues Verfahren zur Herstellung von Polyen(di)aldehyden aus acetalisierten Polyen(di)aldehyden kürzerer Kettenlänge durch eine säurekatalysierte Kondensationsreaktion mit Alkoxydienen.

Lewissäure-katalysierte Additionen von α,β-ungesättigten Ethern (Enolethern) an Acetale sind schon lange bekannt und gehen auf die Arbeiten von Müller-Cunradi und Pieroh zurück (siehe US-Patentschrift 2.165.962). Hoaglin und Hirsch [J.A.C.S. 71, 3468 (1949)] haben diese Reaktion weiter untersucht und die Anwendungsmöglichkeiten erweitert, was ebenfalls Isler et al. in den fünfziger Jahren getan haben, und zwar auf die Synthese von β-Carotin, Crocetindialdehyd, Lycopin sowie β-Apocarotinoiden [siehe Helv. Chim. Acta 39, 249 ff. und 463 ff. (1956), ibid. 42, 854 ff. (1959) sowie US-Patentschriften 2.827.481 und 2.827.482]. Später erweiterte Mukaiyama [Angew. Chem. 89, 858 ff. (1977) und Org. Reactions 28, 203 ff. (1982)] die Reaktion durch Verwendung der gut und leicht zugänglichen Trimethylsilylenolether.

Ueber die ersten Lewissäure-katalysierten Kondensationen von 1-Alkoxy-1,3-dienen (Dienolethern) mit α,β-ungesättigten Acetalen wurde von Nazarov und Krasnaya [J. Gen. Chem. USSR 28, 2477 ff. (1958)] und von Makin [Pure & Appl. Chem. 47, 173 ff. (1976), J. Gen. Chem. USSR 31, 3096 ff. (1961) und 32, 3112 ff. (1962)] berichtet. Dabei erfolgt die Kopplung des Acetals an den Dienolether soweit ersichtlich ausschliesslich an dessen γ-Stellung unter Bildung eines kettenverlängerten α,β-ungesättigten Acetals, das jedoch in Konkurrenz zum ersten Acetal mit weiterem Dienolether reagiert unter Bildung eines weiteren, kettenverlängerten α,β-ungesättigten Acetals usw. [Telomerbildung; siehe auch noch Chemla et al., Bull. Soc. Chim. Fr. 130, 200 ff. (1993)]. Aus diesem Grund ist eine derartige Kondensation für synthetische Zwecke, speziell für die Synthese von Apocarotinoiden, als nicht brauchbar befunden worden [Isler et al., Adv. Org. Chem. 4, 115 ff. (1963)].

Nicht nur 1-Alkoxy-1,3-diene, sondern auch Trimethylsilyloxydiene [vom Typ $CH_2=CH-CH=CH-OSi(CH_3)_3$] können in Gegenwart von Lewissäure-Katalysatoren mit α,β-ungesättigten Acetalen kondensiert werden, wie dies Mukaiyama et al. in Chem. Lett. 1975, 319 ff. offenbarten. Auch bei dieser Kopplung scheint der Angriff ausschliesslich am endständigen (γ-) Kohlenstoffatom des Diensystems zu erfolgen, um "γ-Produkte" zu bilden [siehe Mukaiyama et al., Bull. Chem. Soc. Jap 50, 1161 ff. (1977) sowie Japanische Patentpublikation (Kokai) 36.645/1977]. Im Gegensatz zur Reaktion mit 1-Alkoxy-1,3-dienen, bei welcher ein α,β-ungesättigtes Acetal anfällt, wird bei der Umsetzung von Trimethylsilyloxydienen mit Acetalen ein Aldehyd gebildet, der mit dem Dien nicht weiterreagieren kann (keine Telomerbildung). Unter Verwendung dieser Methode konnten Mukaiyama et al. Vitamin A synthetisieren [siehe Kokai 36.645/1977, Chem. Lett. 1975, 1201 ff. sowie Bull. Chem. Soc. Japan 51, 2077 ff. (1978)] und Mitarbeiter von Rhône-Poulenc neue Zugänge zu Carotinoiden und Vitamin A (siehe DOS 2.701.489 und A.E.C. Société de Chimie Organique et Biologique Nr. 7824350) entwickeln.

Die obenerwähnte, auf den Arbeiten von Nazarov und Krasnaya, Makin sowie Chemla et al. basierende Lewissäure-katalysierte Kondensation eines Dienolethers mit einem α,β-ungesättigten Acetal würde einen sehr wertvollen Zugang zu Apocarotinalen und Bis-apocarotinalen eröffnen, falls die Ausbeute am gewünschten Primärprodukt des Typs $...CH=CH-CH(OAlkyl^1)-CH_2-CH=CH-CH(OAlkyl^1)(OAlkyl^2)$ erhöht bzw. die Telomerbildung unterdrückt werden könnte. So könnte aus diesem Primärprodukt durch Hydrolyse der Acetalgruppe $C(OAlkyl^1)(OAlkyl^2)$ und Elimination von $Alkyl^1OH$ der gewünschte Polyenaldehyd des Typs ...CH=CH-CH=CH-CH=CH-CHO erhalten werden. Zusätzlich zu der Situation, dass bei dieser Reaktion die Bildung der Doppelbindung unter katalytischen Bedingungen erfolgt, werden keine phosphor-, silizium- bzw. schwefelhaltigen Reagenzien benötigt.

Ziel der vorliegenden Erfindung ist es, ausgehend von Polyenacetalen oder -diacetalen kettenverlängerte Polyenaldehyde bzw. -dialdehyde herzustellen, und zwar unter möglichst weitgehender Vermeidung der obenerwähnten Nachteile des Standes der Technik sowie mit Ersatz der bisher zu diesem Zweck verwendeten Wittig-, Horner- oder Julia-Reaktion. Dieses Ziel wird erfindungsgemäss erreicht, indem man ein Polyen-O,O-dialkylacetal oder -di(O,O-dialkylacetal) mit einem 1-Alkoxy-1,3-dien in Gegenwart eines geeigneten Katalysators, nämlich einer Brönstedsäure, zum entsprechenden kettenverlängerten δ-Alkoxy-α,β-ungesättigten Polyenaldehyd bzw. di(δ-alkoxy-a,ß-ungesättigten) Polyendialdehyd in Form dessen Acetals bzw. Diacetals umsetzt, dieses (Di-)Acetal zum entsprechenden (Di-)Aldehyd hydrolysiert und schliesslich vom so gebildeten (Di-)Aldehyd unter basischen oder sauren Bedingungen das δ-ständige Alkanol eliminiert, um den erwünschten (konjugierten) Polyen(di)aldehyd zu erhalten. Nicht nur ist die Reaktion des 1-Alkoxy-1,3-diens mit dem Polyen-O,O-dialkylacetal bzw. -di(O,O-dialkylacetal) neu, sondern es erfolgt dabei ein (soweit ersichtlich) ausschliesslicher Angriff an der γ-Stellung des Alkoxydiens. Durch die sich der Hydrolyse anschliessende basen- induzierte Elimination des Alkanols wird eine (konjugierte) C-C-Doppelbindung gebildet, ohne dass dazu ein phosphor-, silizium- oder schwefelhaltiges Reagens benötigt wird, was im Gegensatz zu der bisher auf diesem Gebiet üblicherweise verwendeten Methodik steht.

Demnach handelt es sich bei der vorliegenden Erfindung um ein Verfahren zur Herstellung eines Polyenaldehyds oder -dialdehyds der allgemeinen Formel

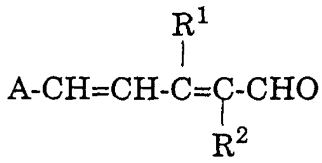 I'

oder

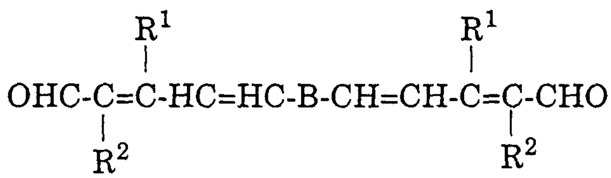 I''

worin

A eine monovalente, gegebenenfalls methylsubstituierte, konjugierte Polyengruppe,
B eine bivalente, gegebenenfalls methylsubstituierte, konjugierte Polyengruppe und
$R^1$ und $R^2$ jeweils Wasserstoff oder Methyl bedeuten, wobei sich die Gruppe(n) $-CH=CH-C(R^1)=C(R^2)-CHO$ jeweils in der(den) Endstellung(en) der konjugierten Kette der Gruppe A bzw. B befindet(n),

das dadurch gekennzeichnet ist, dass man ein Polyen-O,O-dialkylacetal oder -di(O,O-dialkylacetal) der allgemeinen Formel

$$A-CH(OR^3)_2 \qquad II'$$

bzw.

$$(R^3O)_2HC-B-CH(OR^3)_2 \qquad II''$$

worin

A und B die oben angegebenen Bedeutungen besitzen, wobei sich in diesem Fall die Gruppe(n) $-CH(OR^3)_2$ jeweils in der(den) Endstellung(en) der konjugierten Kette der Gruppe A bzw. B befindet(n) und
$R^3$ $C_{1-6}$-Alkyl bedeutet,

mit einem 1-Alkoxy-1,3-dien der allgemeinen Formel

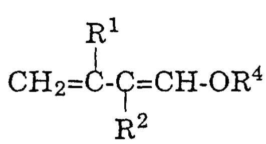 III

worin

$R^1$ und $R^2$ die oben angegebenen Bedeutungen besitzen und
$R^4$ $C_{1-6}$-Alkyl bedeutet,

in Gegenwart einer Brönstedsäure umsetzt, das Reaktionsgemisch hydrolysiert, und von der so hergestellten Verbindung der allgemeinen Formel

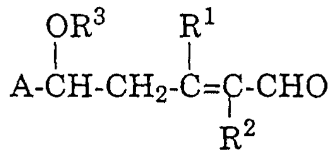

IV'

bzw.

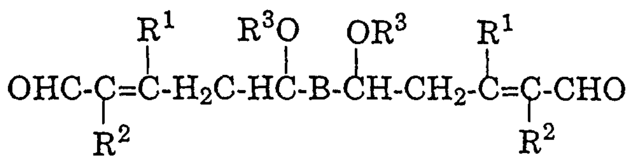

IV''

worin A, B, $R^1$, $R^2$ und $R^3$ die oben angegebenen Bedeutungen besitzen, wobei sich in diesem Fall die Gruppe (n) $-CH(OR^3)-CH_2-C(R^1)=C(R^2)-CHO$ jeweils in der(den) Endstellung(en) der konjugierten Kette der Gruppe A bzw. B befindet(n),
unter basischen Bedingungen in Gegenwart eines Alkalimetallalkoholat als Base den Alkohol $R^3OH$ abspaltet.

Das erfindungsgemässe Verfahren kann im Prinzip bei allen obenerwähnten Polyen-O,O-dialkylacetalen der Formel II' oder Polyen-di(O,O-dialkylacetalen) der Formel II", welche am Ende bzw. an beiden Enden der Polyenkette die Acetalgruppe $-CH(OR^3)_2$ aufweisen, Verwendung finden. Unter solchen Edukten befinden sich u.a. die folgenden Unterklassen [wobei die in der Carotinoid-Chemie übliche (mit einfachen Strichen) abgekürzte Darstellungsweise für die Strukturformeln benutzt wird]:

Alicyclisch-aliphatische Polyen-O,O-dialkylacetale, die hauptsächlich dem Gebiet der Carotinoide [als Acetale asymmetrischer Carotinoid-Aldehyde mit einem sechsgliedrigen (Cyclohexen-)Ring] angehören, der allgemeinen Formel

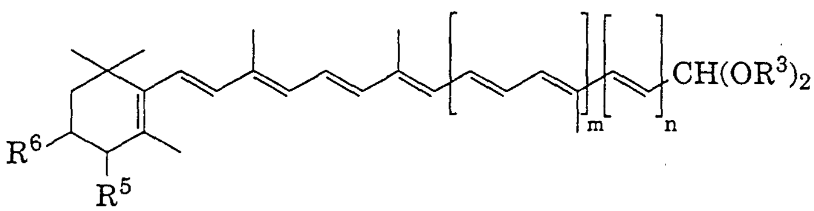

IIa

worin

| | |
|---|---|
| $R^3$ | die oben angegebene Bedeutung besitzt und |
| $R^5$ und $R^6$ | unabhängig voneinander Wasserstoff, eine gegebenenfalls geschützte Hydroxygruppe oder eine gegebenenfalls geschützte Oxogruppe, |
| m | 0, 1, 2, 3 oder 4 und |
| n | 0 oder 1 bedeuten, |

welche nach Durchführung des erfindungsgemässen mehrstufigen Verfahrens in die entsprechenden alicyclisch-aliphatischen Polyenaldehyde der allgemeinen Formel

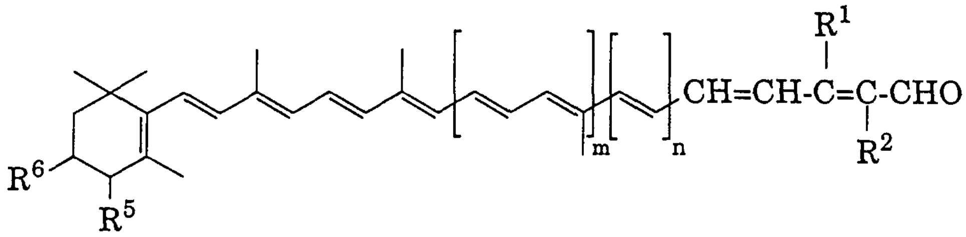

Ia

übergeführt werden;

Aliphatische Polyen-O,O-dialkylacetale, die ebenfalls hauptsächlich dem Gebiet der Carotinoide (als Acetale offenkettiger asymmetrischer Carotinoid-Aldehyde) angehören, der allgemeinen Formel

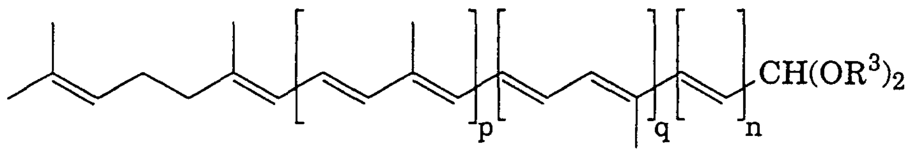

IIb

worin

$R^3$ die oben angegebene Bedeutung besitzt und
p 0, 1 oder 2,
q 0, 1, 2 oder 3 und
n 0 oder 1 bedeuten,

welche nach Durchführung des erfindungsgemässen mehrstufigen Verfahrens in die entsprechenden aliphatischen Polyenaldehyde der allgemeinen Formel

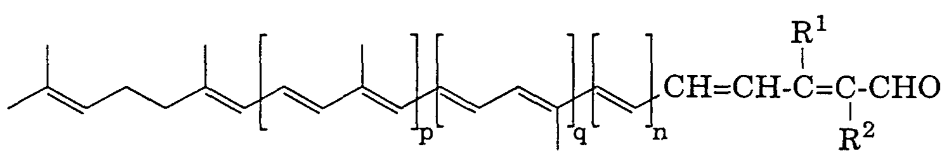

Ib

übergeführt werden;

Aliphatische Polyen-di(O,O-dialkylacetale), die ebenfalls hauptsächlich dem Gebiet der Carotinoide (als Acetale symmetrischer Carotinoid-Dialdehyde) angehören, der allgemeinen Formel

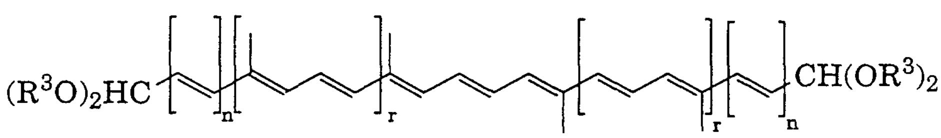

IIc

worin

$R^3$ die oben angegebene Bedeutung besitzt und
r 0, 1 oder 2 und
n 0 oder 1 bedeuten,

welche nach Durchführung des erfindungsgemässen mehrstufigen Verfahrens in die entsprechenden aliphatischen Polyendialdehyde der allgemeinen Formel

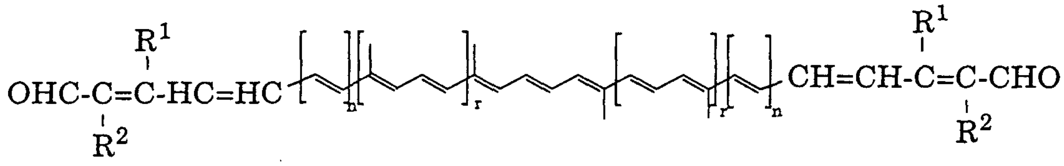 Ic

übergeführt werden.

Die Edukte der allgemeinen Formeln IIa, IIb und IIc können durch die allgemeine Formel II umfasst werden:

$$R - CH(OR^3)_2 \qquad II$$

worin

R eine Gruppe (a), (b) oder (c)

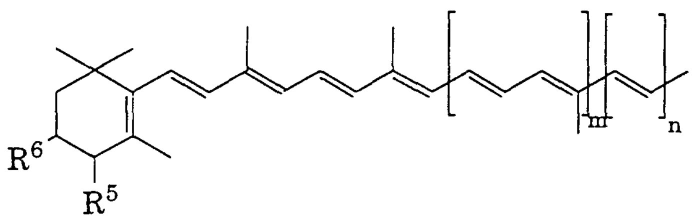 (a)

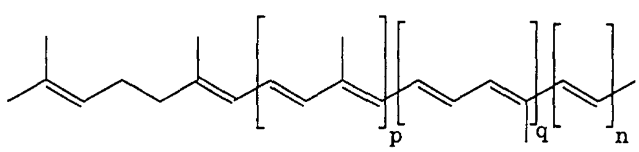 (b)

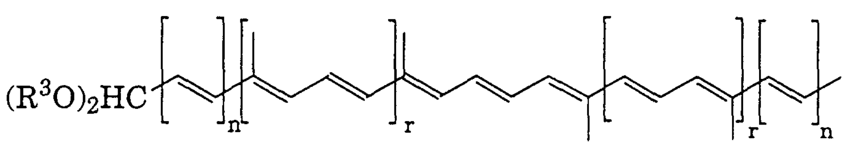 (c)

bedeutet und

$R^3$, $R^5$, $R^6$, m, n, p, q und r die oben angegebenen Bedeutungen besitzen.

Nach Durchführung des erfindungsgemässen mehrstufigen Verfahrens wird das Edukt der Formel II in das entsprechende Produkt der Formel I übergeführt:

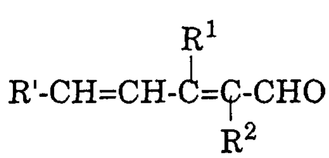 I

worin R' eine Gruppe (a) oder (b) bzw. eine Gruppe (c')

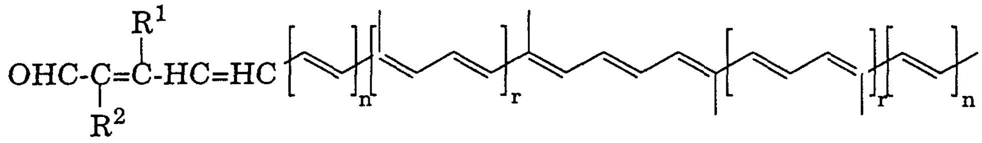

(c')

bedeutet.

Die Formel I umfasst dann die Formeln Ia, Ib und Ic.

Falls das Produkt der Formel I, insbesondere der Formel Ia, am Cyclohexenring eine oder zwei geschützte Gruppen ($R^5$, $R^6$) aufweist, kann man gewünschtenfalls die vorhandene(n) Schutzgruppe(n) abspalten, was einen weiteren Aspekt der vorliegenden Erfindung darstellt.

Im Rahmen der vorliegenden Erfindung umfasst der Ausdruck "$C_{1-6}$-Alkyl" geradkettige und verzweigte Gruppen, wie beispielsweise Methyl, Ethyl und Isobutyl.

Der Ausdruck "geschützte Hydroxygruppe" umfasst übliche (besonders auf dem Gebiet der Carotinoide geläufige) geschützte Hydroxygruppen, insbesondere verätherte Hydroxygruppen und Acyloxygruppen. Bei den "verätherten Hydroxygruppen" handelt es sich beispielsweise um $C_{1-5}$-Alkoxygruppen, vorzugsweise Methoxy und Ethoxy; $C_{2-16}$-Alkoxyalkoxygruppen, vorzugsweise 1-Methoxy-1-methylethoxy; Arylalkoxygruppen, vorzugsweise Benzyloxy; Tetrahydropyranyloxy; und Tri($C_{1-5}$-alkyl)silyloxygruppen, vorzugsweise Trimethylsilyloxy. Die Acyloxygruppen umfassen insbesondere Alkanoyloxy- und Aroyloxygruppen mit bis zu 8 Kohlenstoffatomen, wie beispielsweise Formyloxy, Acetoxy, Propionyloxy und Benzoyloxy.

Der Ausdruck "geschützte Oxogruppe" umfasst ebenfalls übliche (besonders auf dem Gebiet der Carotinoide geläufige) geschützte Oxogruppen. Bevorzugt sind die acetalisierten Oxogruppen, insbesondere diejenigen, worin der Ausdruck geschützte Oxogruppe für zwei $C_{1-5}$-Alkoxygruppen (z.B. für zwei Methoxygruppen) oder für eine $C_{2-6}$-Alkylendioxygruppe (z.B. Ethylendioxy oder 2,3-Butylendioxy) steht. Ferner kann eine Oxogruppe auch als Enolether geschützt sein, und zwar vor allem im Falle von $\alpha$-Hydroxyketonen (z.B. $R^5$ und $R^6$ bedeuten Hydroxy resp. Oxo oder umgekehrt), wobei die Verätherung des Endiols vorzugsweise auch durch Bildung eines cyclischen Acetals oder Ketals (z.B. mit Aceton zum Acetonid) erfolgen kann. Die Oxogruppe kann auch beispielsweise als ein Imin geschützt sein.

Die im Rahmen der vorliegenden Erfindung offenbarten Formeln von Polyenen umfassen jeweils isomere Formen, z.B. optisch aktive und cis/trans bzw. E/Z-Isomere, sowie Gemische hiervon, sofern nicht ausdrücklich anders erwähnt. Als Beispiel eines chiralen (optischen aktiven) Zentrums sei erwähnt das den Rest $R^5$ oder $R^6$ tragende Kohlenstoffatom, falls $R^5$ bzw. $R^6$ eine gegebenenfalls geschützte Hydroxygruppe bedeutet (siehe Formeln Ia und IIa). Was die E/Z-Isomerie anbelangt, so sind im allgemeinen die (all-E)-Isomeren der Edukte und der Produkte des erfindungsgemässen Verfahrens bevorzugt.

Der erste Verfahrensschritt des erfindungsgemässen Verfahrens wird zweckmässigerweise durchgeführt, indem man das Polyen-(di-)O,O-dialkylacetal der Formel II' oder II" mit dem 1-Alkoxy-1,3-dien der Formel III in einem organischen Lösungsmittel bei Temperaturen im Bereich von -60°C bis +60°C, vorzugsweise im Temperaturbereich von -20°C bis zur Raumtemperatur, und in Gegenwart einer Brönstedsäure umsetzt. Als organische Lösungsmittel eignen sich im allgemeinen alle aprotischen polaren oder unpolaren Lösungsmittel. Bevorzugte derartige Lösungsmittel sind niedere aliphatische und cyclische Kohlenwasserstoffe, z.B. n-Pentan, n-Hexan und Cyclohexan; niedere, halogenierte aliphatische Kohlenwasserstoffe, z.B. Methylenchlorid und Chloroform; niedere aliphatische und cyclische Ether, z.B. Diethylether, tert.Butylmethylether und Tetrahydrofuran; niedere aliphatische Nitrile, z.B. Acetonitril; sowie Aromaten, z.B. Toluol. Besonders bevorzugte Lösungsmittel sind n-Hexan und Toluol. Beispiele der verwendbaren Brönstedsäuren sind p-Toluolsulfonsäure, Methansulfonsäure, Trifluormethansulfonsäure, Schwefelsäure sowie Trifluoressigsäure. Diese werden im allgemeinen in katalytischen Mengen eingesetzt, und zwar zweckmässigerweise in einer Menge, die zwischen 0,5 und 5 Molprozent bezogen auf die eingesetzte Menge des Polyen-(di-)O,O-dialkylacetals beträgt, und vorzugsweise im Molprozentbereich von 1% bis 2% liegt. Zudem verwendet man zweckmässigerweise 1,05 bis 1,6 Aequivalente 1-Alkoxy-1,3-dien pro Aequivalent Polyen-O,O-dialkylacetal oder 2,1 bis 3,2 Aequivalente 1-Alkoxy-1,3-dien pro Aequivalent Polyen-di(O,O-dialkylacetal), vorzugsweise etwa 1,2 bis etwa 1,4 bzw. etwa 2,4 bis etwa 2,8 Aequivalente. Ausserdem erfolgt die Umsetzung zweckmässigerweise bei Normaldruck, wobei im allgemeinen der Druck nicht kritisch ist.

Gewünschtenfalls könnte das Zwischenprodukt (die Verbindung der Formel IV' oder IV" in Form ihres Dialkylacetals) aus dem Reaktionsgemisch isoliert und anschliessend zur entsprechenden Verbindung der Formel IV' bzw. IV" hydrolysiert werden. Allerdings erweist es sich als zweckmässiger, keine derartige Isolierung und nachfolgende Hydrolyse vorzunehmen, sondern unmittelbar nach Beendigung der Reaktion II'/II" + III das Zwischenprodukt im Reaktionsgemisch selber zu hydrolysieren, um in diesen Fällen zur Verbindung der Formel IV' oder IV" (nachfolgend als

IV'/IV" abgekürzt) zu gelangen. Die Hydrolyse kann geeigneterweise erfolgen, indem man zum Reaktionsgemisch eine wässrige Lösung einer schwachen Säure, vorzugsweise leicht verdünnte wässrige Essigsäure, gibt und das Gemisch anschliessend eine Weile, beispielsweise etwa 30 Minuten bis etwa 2 Stunden, rührt, und zwar zweckmässigerweise im Temperaturbereich von 0°C bis zur Raumtemperatur.

Das jeweilige Produkt der Formel IV' oder IV" kann in an sich bekannter Weise vom Reaktionsgemisch isoliert und gewünschtenfalls gereinigt werden. Typischerweise wird das Gemisch mit Wasser vereinigt und das Ganze mit einem mit Wasser nicht mischbaren organischen Lösungsmittel, wie beispielsweise mit einem niederen Alkan, Dialkylether oder aliphatischen Ester, z.B. n-Hexan, tert.Butylmethylether bzw. Ethylacetat, extrahiert und die organische Phase mit Wasser und/oder gesättigter wässriger Natriumchlorid- und/oder Natriumbicarbonat-Lösung gewaschen, getrocknet und eingeengt. Eine Alternative besteht darin, dass man dem Reaktionsgemisch Wasser zugibt und das daraus resultierende ausgefallene Rohprodukt abfiltriert, wie im Falle des Extraktes wäscht und dann trocknet. Das so isolierte und zumindest einigermassen gewaschene Rohprodukt kann dann gewünschtenfalls weitergereinigt werden, beispielsweise durch Säulenchromatographie, z.B. unter Verwendung von solchen Eluierungsmitteln als n-Hexan, Ethylacetat, Toluol oder Gemischen davon, oder (Um)Kristallisation, beispielsweise aus einem Alkohol, z.B. Methanol oder Ethanol. Alternativ, und oft bevorzugt, kann das beispielsweise in ein niederes Alkanol aufgenommene Rohprodukt unmittelbar im letzten Verfahrensschritt der vorliegenden Erfindung umgesetzt werden, und zwar im Rahmen eines "Durchprozesses" II'/II" + III → IV'/IV" → I'/I".

Was den letzten Verfahrensschritt, d.h. die Abspaltung des Alkohols $R^3OH$ von der Verbindung der Formel IV' oder IV", anbelangt, so sind Eliminierungen des Alkohols aus β-Alkoxyaldehyden oder δ-Alkoxy-α,β-ungesättigten Aldehyden unter Bildung der entsprechenden α,β-ungesättigten bzw. α,β,γ,δ-ungesättigten Aldehyde in der Fachliteratur bekannt und können unter verschiedenen Bedingungen durchgeführt werden. Beispielsweise wird im Rahmen bekannter baseninduzierter Eliminierungen als Base sehr oft 1,8-Diazabicyclo[5.4.0]undec-7-en verwendet, und zwar in einer Menge von etwa 2 bis 4 Aequivalenten bezogen auf die Menge eingesetzten Aldehyds. Es werden solche Bedingungen bei der bekannten Herstellung von Carotinoiden [siehe u.a. Bull. Chem. Soc. Japan 50, 1161 ff (1977), ibid. 51, 2077 ff. (1978), Chem. Lett. 1975, 1201 ff. und Deutsche Offenlegungsschrift 2.701.489] und von Vitamin A (siehe u.a. Chem. Lett. 1975, 1201 ff.) verwendet. Auch Aluminiumoxid ist bei der Herstellung von Vitamin A durch Alkoholabspaltung verwendet worden [J. Gen. Chem. USSR 32, 63 ff. (1962)]. Als Beispiele von säureinduzierten Alkoholabspaltungen wird nochmals auf Bull. Chem. Soc. Japan 50, 1161 ff. (1977) sowie auf J. Gen. Chem. USSR 30, 3875 ff. (1960) verwiesen, bei denen p-Toluolsulfonsäure bzw. 85%ige Phosphorsäure als saurer Katalysator verwendet wird. Besonders bei der Herstellung von Carotinoiden ist für eine derartige Abspaltung das Puffersystem Natriumacetat/Essigsäure [Helv. Chem. Acta. 39, 249 ff. und 463 ff. (1956) und US-Patentschriften 2.827.481 und 2.827.482] oder Natriumformiat/Ameisensäure [Synthesis 1981, 137 ff.] eingesetzt worden.

Im Falle des erfindungsgemässen Verfahrens werden basische Bedingungen verwendet, wobei als Base ein Alkalimetallalkoholat verwendet wird.

Darüber hinaus kann die Abspaltung des Alkohols $R^3OH$ auch nur mit katalytischen Mengen der Base, d.h. mit weniger als einem Aequivalent bezogen auf ein Aequivalent der Verbindung der Formel IV' oder IV", durchgeführt werden. So wird der letzte Verfahrensschritt in diesem Fall zweckmässigerweise durchgeführt, indem man die in einem geeigneten organischen Lösungsmittel gelöste Verbindung der Formel IV' oder IV" in Gegenwart einer katalytischen Menge der Base unter Abspaltung des Alkohols $R^3OH$ zum entsprechenden Polyen(di)aldehyd der Formel I' bzw. I" umsetzt. Als organische Lösungsmittel eignen sich im allgemeinen protische, aprotische oder Gemische davon, wie beispielsweise Alkohole und Alkoholgemische; bzw. Aromaten, z.B. Toluol; und niedere aliphatische Ester, z.B. Ethylacetat. Es eignen sich als Basen Alkalimetallalkoholate, wie beispielsweise Natriummethylat, Natriumethylat, Kaliummethylat, Kaliumethylat und Kalium-tert.butylat. Wie oben erwähnt, verwendet man zweckmässigerweise höchstens ein Aequivalent der Base pro Aequivalent der Verbindung der Formel IV' oder IV", vorzugsweise 0,05 bis 0,3 Aequivalent. Die Umsetzung erfolgt geeigneterweise im Temperaturbereich von -20°C bis 100°C, vorzugsweise bei Temperaturen von 0°C bis 50°C. Ausserdem erfolgt die Umsetzung zweckmässigerweise bei Normaldruck, wobei im allgemeinen der Druck nicht kritisch ist.

Es hat sich als besonders vorteilhaft erwiesen den letzten Verfahrensschritt unter Verwendung eines Natriumalkoxids als Alkalimetallalkoholat und des entsprechenden Alkanols als Lösungsmittel bei Temperaturen zwischen -20°C und der Rückflusstemperatur des jeweiligen Reaktionsgemisches, vorzugsweise im Temperaturbereich von 0°C bis 40°C, durchzuführen. Dabei wird zweckmässigerweise im voraus entweder eine Lösung des Natriumalkoxids im Alkanol bereitgestellt, oder man stellt diese Lösung frisch aus metallischem Natrium und dem Alkanol her. Das Zusammenbringen der alkanolischen Lösung des Natriumalkoxids mit der vorzugsweise ebenfalls im voraus vorbereiteten Lösung der Verbindung der Formel IV'/IV" in (dem gleichen) Alkanol kann in beliebiger Reihenfolge und vorzugsweise bei Raumtemperatur erfolgen. Man rührt dann das Reaktionsgemisch, und die Reaktion ist normalerweise spätestens nach einer bis drei Stunden beendet.

Ungeachtet der gewählten Prozedur des letzten Verfahrensschrittes kann das Produkt in an sich bekannter Weise vom Reaktionsgemisch isoliert und gereinigt werden. Die jeweilige Aufarbeitung beinhaltet normalerweise das

Neutralisieren der verbleibenden Base, und zwar durch Zugabe einer organischen oder anorganischen Säure, wie beispielsweise einer Carbonsäure, z.B. Essigsäure, bzw. einer wässrigen Mineralsäure, z.B. verdünnte Schwefelsäure.

Im besonderen Fall der oben beschriebenen Prozedur mit dem Natriumalkoxid als Base wird nach Beendigung der Reaktion das Gemisch zweckmässigerweise auf Raumtemperatur oder sogar bis auf etwa 0°C abgekühlt und danach vorzugsweise mit wässriger Essigsäure neutralisiert. Die Auskristallisation des Produktes der Formel I' oder I" kann auch noch durch weiteres Abkühlen gefördert werden. Nach dessen Isolierung, geeigneterweise durch Abfiltration, kann das Produkt gewaschen, beispielsweise mit Wasser und/oder wässrigem Alkohol, und schliesslich gegebenenfalls unter vermindertem Druck getrocknet werden. Gewünschtenfalls können solche weitere Methoden wie Säulenchromatographie und Umkristallisation eingesetzt werden, um zu einem noch reineren Produkt zu gelangen.

Im erhaltenen Produkt der Formel I' oder I" gegebenenfalls vorhandene Schutzgruppen ($R^5$ und/oder $R^6$ als geschützte Hydroxy- oder Oxogruppe) können gewünschtenfalls nach an sich bekannten Methoden, z.B. durch Hydrolyse mit Säure oder Base, abgespalten werden.

In dem oben definierten erfindungsgemässen Verfahren bedeuten R vorzugsweise entweder eine Gruppe (a), in der $R^5$ und $R^6$ beide Wasserstoff und n O bedeuten, oder eine Gruppe (c), in der beide n O bedeuten, und in beiden Fällen $R^1$ und $R^2$ vorzugsweise Wasserstoff resp. Methyl.

Wie oben erwähnt, besteht bei der Durchführung des erfindungsgemässen Verfahrens gegenüber dem Stande der Technik (insbesondere den obenerwähnten Arbeiten von Nazarov und Krasnaya, Makin sowie Chemla et al.) der Vorteil darin, dass u.a. die Telomerbildung weitgehend unterdrückt wird. Obwohl bei dem erfindungsgemässen Verfahren die Telomerbildung als Folge der weiteren Reaktion der Verbindung der Formel IV' oder IV" in Acetalform mit dem 1-Alkoxy-1,3-dien der Formel III nicht immer vollständig unterdrückt werden kann, ist dies schliesslich weit weniger gravierend als erwartet. Die nach der zwischenstufigen Hydrolyse erfolgende Abspaltung des Alkohols $R^3OH$ von der Verbindung der Formel IV' bzw. IV" kann nämlich ohne weiteres in Gegenwart eines in relativ kleiner Menge als Nebenprodukt mithergestellten Telomers, z.B. der Formel $A\text{-}CH(OR^3)\text{-}CH_2\text{-}C(R^1)=C(R^2)\text{-}CH(OR^3)\text{-}CH_2\text{-}C(R^1)=C(R^2)\text{-}CHO$ bzw. $OHC\text{-}(R^2)C=(R^1)C\text{-}H_2C\text{-}(R^3O)HC\text{-}(R^2)C=(R^1)C\text{-}H_2C\text{-}(R^3O)HC\text{-}B\text{-}CH(OR^3)\text{-}CH_2\text{-}C(R^1)=C(R^2)\text{-}CH(OR^3)\text{-}CH_2\text{-}C(R^1)=C(R^2)\text{-}CHO$ (jeweils einmalige weitere Reaktion), erfolgen, wobei unter den speziell angewandten Reaktionsbedingungen auch eine analoge Alkoholabspaltung von dem Telomer stattfindet. Die letztere Abspaltung erfolgt jedoch unvollständig, indem praktisch nur der von der endständigen Aldehydgruppe am nächsten gelegene Alkohol $R^3OH$ (δ-Alkoxy) abgespalten wird. Das Ergebnis dieser unvollständigen Alkoholabspaltung vom Telomer besteht darin, dass das erwünschte Produkt der Formel I' bzw. I" vom in diesem Stadium vorliegenden Nebenprodukt viel leichter entfernt werden kann als wenn alle Alkoxygruppen $OR^3$ vom Telomer abgespalten würden. So bleibt nämlich das Nebenprodukt, welches noch einen oder mehrere Substituenten $OR^3$ aufweist, in der Mutterlauge des Reaktionsgemisches, während das erwünschte Produkt auskristallisiert und demzufolge leicht zu entfernen ist, z.B. durch Filtration. Dass eben das Telomer nur den (jeweiligen) δ-ständigen Alkohol $R^3OH$ bei dem Abspaltungsschritt des erfindungsgemässen Verfahrens verliert, stellt ein völlig überraschendes Ergebnis dar.

Während einige der Edukte des erfindungsgemässen Verfahrens bekannt sind, sind andere aus zum Teil bekannten Vorstufen nach an sich bekannten Methoden herstellbar.

So können beispielsweise die Polyen-O,O-dialkylacetale der Formel II' und die Polyen-di(O,O-dialkylacetale) der Formel II" sehr einfach auf bekannte allgemeine Weise durch Umsetzung des Polyen-monoaldehyds der Formel A-CHO bzw. Polyen-dialdehyds der Formel OHC-B-CHO mit dem diesbezüglichen Orthoameisensäure-trialkylester hergestellt werden, insbesondere in dem entsprechenden $C_{1\text{-}6}$-Alkanol, z.B. Methanol für das O,O-Dimethylacetal, und in Gegenwart einer katalytischen Menge einer organischen Säure oder einer Lewissäure, z.B. p-Toluolsulfonsäure bzw. Zinkchlorid [siehe beispielsweise Organikum, Organisch-chemisches Grundpraktikum, 6. Auflage, S. 377 ff. (1963)]. Die Reaktion verläuft in Suspension, d.h. der jeweilige Polyen-monoaldehyd oder -dialdehyd wird im Alkanol suspendiert, und dann werden zweckmässigerweise etwa zwei bzw. vier Moläquivalente des Orthoameisensäure-trialkylesters zur Suspension gegeben, gefolgt von einer Spur sauren Katalysators, z.B. p-Toluolsulfonsäure. Dabei löst sich der Mono- oder Dialdehyd langsam auf, und das gebildete Polyen-O,O-dialkylacetal bzw. -di(O,O-dialkylacetal) der Formel II'/II" kristallisiert gleichzeitig langsam aus. Die Umsetzung wird zweckmässigerweise im Temperaturbereich von etwa 0°C bis etwa 40°C durchgeführt und dauert in der Regel von etwa 2 bis etwa 4 Stunden. Als weitere Literaturstellen, welche die allgemein bekannte Acetalisierungsmethode veranschaulichen, wird auf die europäischen Patentpublikationen 252 389 und 391 033 sowie auf J. Mol. Cat. 79, 117 ff. (1993) verwiesen.

Die Polyen-monoaldehyde A-CHO und -dialdehyde OHC-B-CHO ihrerseits sind entweder bekannt, insbesondere aus der Fachliteratur betreffend Carotinoide, oder - falls neu - können nach an sich bekannten einschägigen Methoden hergestellt werden. So sind beispielsweise die Umsetzung von verschiedenen $C_{15}$-Wittigsalzen mit 2,7-Dimethyl-2,4,6-octatriendial (dem sogenannten "$C_{10}$-Dialdehyd") zu den entsprechenden Monoaldehyden, die Umsetzung von verschiedenen $C_5$-Wittigaldehyden mit langkettigen Polyenaldehyden ebenfalls zu solchen Monoaldehyden sowie die zweifache Umsetzung des $C_{10}$-Dialdehyds mit $C_5$- oder $C_{10}$-Wittigaldehyden zu verschiedenen Dialdehyden aus dieser Literatur bekannt geworden. Das Lehrbuch "Carotinoids" (O. Isler, Birkhäuser Verlag Basel und Stuttgart, 1971), besonders dessen Kapitel VI und XII und die darin erwähnte weitere Literatur, liefert viele nützliche Hinweise

auf die Herstellung und das Vorkommen der bekannten Mono- und Dialdehyde. Falls Edukte eingesetzt werden, welche geschützte Hydroxy-, Oxo- bzw. Formylgruppen aufweisen, so können solche "geschützte" Edukte beispielsweise unmittelbar aus den entsprechenden ungeschützten nach an sich bekannten Methoden hergestellt werden.

Die 1-Alkoxy-1,3-diene der Formel III sind zum Teil bekannte Verbindungen; die restlichen (neuen) können aus bekannten Ausgangsmaterialien nach an sich bekannten Methoden hergestellt werden.

So ist beispielsweise das 1-Ethoxy-2-methyl-1,3-butadien (der Formel III, worin $R^1$ Wasserstoff, $R^2$ Methyl und $R^4$ Ethyl bedeuten) aus der Literatur seit langem bekannt [siehe u.a. J.A.C.S. 91, 3281 ff. (1969), Bull. Soc. Chim. Fr. 1963, 1646 ff., sowie J. Gen. Chem. USSR 29, 3649 ff. (1959)] und wurde jeweils durch zweimalige Abspaltung von Ethanol aus 1,1,3-Triethoxy-2-methyl-butan hergestellt. Das Butan seinerseits kann durch eine seit langem bekannte Enoletherkondensation (siehe US-Patentschrift 2.165.962) aus den beiden gut zugänglichen Ausgangsmaterialien Acetaldehyd-diethylacetal und Ethyl-(1-propenyl)-ether hergestellt werden [siehe zudem J.A.C.S. 71, 3468 ff. (1949) sowie J. Gen. Chem. USSR 29, 3641 ff. (1959)]. Hierbei erwärmt man leicht etwa 2 bis 3 Aequivalente des Acetals pro Aequivalent Ethylpropenylether bis etwa 2 Stunden bei etwa 35°C mit etwa 0,2 Molprozent Bortrifluoridetherat als Katalysator ohne Lösungsmittel und erhält das erwünschte Butan in einer etwa 66%igen Ausbeute. Die anschliessende zweimalige Abspaltung von Ethanol aus dem 1,1,3-Triethoxy-2-methyl-butan wurde gemäss dem einschlägigen Stand der Technik auf zwei verschiedenen Wegen realisiert:

(i) durch Abspaltung in der Flüssigphase, indem man das 1,1,3-Triethoxy-2-methyl-butan in Isochinolin enthaltend eine katalytische Menge p-Toluolsulfonsäure bei etwa 220°C zutropft und das sofort gebildete 1-Ethoxy-2-methyl-1,3-butadien abdestilliert. Die Ausbeuten sind bei dieser in Bull. Soc. Chim. Fr. 1963, 1646 ff. beschriebenen Methode allerdings mittelmässig (etwa 40-50%); oder

(ii) durch Abspaltung in der Gasphase bei 300-350°C unter Vakuum an einem sauren Katalysator, z.B. Mononatrium-, Magnesiumdihydrogen- oder Monoammoniumphosphat [J. Gen. Chem. USSR 29, 3649 ff. (1959)], oder basischen Katalysator, z.B. Aluminiumoxid (US-Patentschrift 2.573.678), wobei die Ausbeuten im Bereich von etwa 50 bis 80% liegen.

Eine Abspaltung von Ethanol in der Gasphase bei erhöhter Temperatur an einem fixierten, festen Katalysator ist bei einem technischen Prozess viel attraktiver als eine Abspaltung in der Flüssigphase, da die erstere Methode u. a. kein Lösungsmittel und eine einfache Reaktionsführung und Aufarbeitung aufweist. Daher ist die in der Gasphase erfolgende Abspaltung bevorzugt.

Auch das 1-Methoxy-2-methyl-1,3-butadien (der Formel III, worin $R^1$ Wasserstoff und $R^2$ und $R^4$ beide Methyl bedeuten) ist aus der Literatur [Japanische Patentpublikation (Kokai) 50891/1989] bekannt. Es kann beispielsweise analog der oben beschriebenen Herstellung von 1-Ethoxy-2-methyl-1,3-butadien ausgehend von Acetaldehyd-dimethylacetal und Methyl-(1-propenyl)-ether über das 1,1,3-Trimethoxy-2-methyl-butan hergestellt werden.

Uebersichtsartikel zur Herstellung der 1-Alkoxy-1,3-diene befinden sich in Russian Chem. Rev. 38, 237 ff. (1969) und in Pure and Appl. Chem. 47, 173 ff. (1976); für weitere Literatur über deren Herstellung durch Gasphasenkatalyse wird auf Lieb. Ann. Chem. 568, 1 ff. (1950), Can. J. Res. B 28, 689 ff. (1950), ibid. B 25, 118 ff. (1947) sowie Chem. Ber. 77, 108 ff. (1944) verwiesen.

Auch die restlichen 1-Alkoxy-1,3-diene der Formel III sind zum Teil bekannte Verbindungen und können analog den obenerwähnten Verbindungen hergestellt werden. Das nachfolgende Reaktionsschema stellt eine Zusammenfassung des oben näher erläuterten mehrstufigen Verfahrens dar, gemäss welchem alle 1-Alkoxy-1,3-diene der Formel III hergestellt werden können:

Reaktionsschema

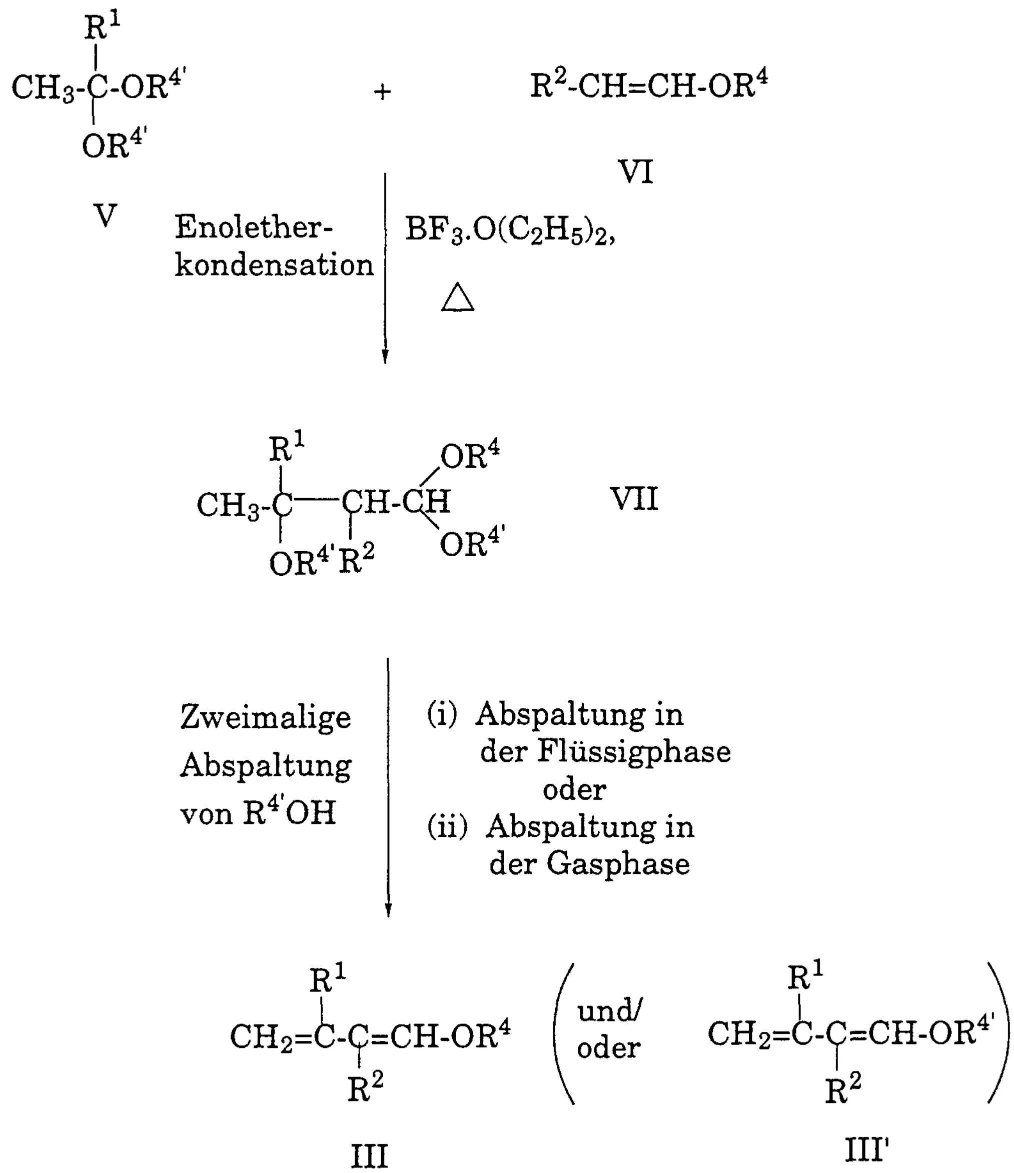


In diesem Reaktionsschema besitzen $R^1$, $R^2$ und $R^4$ die oben angegebenen Bedeutungen; $R^{4'}$ bedeutet $C_{1-6}$-Alkyl, und zwar zweckmässigerweise - um Produktegemische zu vermeiden - das gleiche Alkyl wie $R^4$. Falls sich $R^{4'}$ von $R^4$ unterscheidet, kann nämlich ein Produktegemisch der beiden Verbindungen III und III' resultieren.

Bei der oben beschriebenen Herstellung der 1-Alkoxy-1,3-diene kann jeweils das Produkt oder Zwischenprodukt auf an sich bekannte Weise isoliert und gereinigt werden.

Viele der als Zwischenprodukte des erfindungsgemässen Verfahrens agierenden Verbindungen der allgemeinen Formeln IV' und IV" sind neue Verbindungen und stellen einen weiteren Aspekt der vorliegenden Erfindung dar. Die neuen, efindungsgemäsen Verbindungen sind diejenigen der allgemeinen Formeln IV' und IV", worin A eine Gruppe (a) oder (b) bzw. B eine Gruppe (c"), wie diese in Anspruch 11 angegeten ist, bedeutet und $R^1$, $R^2$, $R^3$, $R^5$, $R^6$, m, n, p, q und r die oben angegebenen Bedeutungen besitzen, mit der Massgabe, dass in der Gruppe (a) m und n nicht gleichzeitig O sind.

Unter diesen neuen Verbindungen der allgemeinen Formeln IV' und IV" befinden sich:

12'-Methoxy-11',12'-dihydro-8'-apo-β-carotinal,
8'-Methoxy-7',8'-dihydro-4'-apo-ß-carotinal,

4'-Methoxy-β,ψ-carotin-16'-al,
11,12,11',12'-Tetrahydro-12,12'-dimethoxy-8,8'-diapocarotin-8,8'-dial sowie
7,8,7',8'-Tetrahydro-8,8'-dimethoxy-4,4'-diapocarotin-4,4'-dial.

Die Endprodukte des erfindungsgemässen Verfahrens, d.h. die Polyenaldehyde und -dialdehyde der allgemeinen Formeln I' und I", gehören grösstenteils dem Gebiet der Carotinoide an und finden entsprechende Verwendung, beispielsweise als Farbstoffe bzw. Pigmente für Lebensmittel, den Eidotter, die Integumente (insbesondere Haut, Ständer und Schnäbel) und/oder das subkutane Fett von Geflügel, das Fleisch und/oder die Integumente (insbesondere Haut, Schuppen und Schale) von Fischen und Crustaceen usw. Diese Verwendung kann nach an sich bekannten Methoden erfolgen, wie diese beispielsweise in der europäischen Patentpublikation Nr. 630.578 beschrieben ist.

Die Erfindung wird anhand der nachfolgenden Beispiele veranschaulicht:

A. Herstellung der Polyen-(di-)O,O-dialkylacetale (Verbindungen der Formeln II' und II")

Beispiel 1

15-Apo-β-carotinal-dimethylacetal(Vitamin A-Aldehyd-dimethylacetal)

In einem mit Magnetrührer, Argonbegasung und Thermometer ausgestatteten 100 ml-Vierhals-Sulfierkolben wurden 2,84 g (10 mmol) Vitamin A-Aldehyd (>99% rein) in 30 ml Methanol und 11 ml (100 mmol, 10 Aeq.) Orthoameisensäure-trimethylester vorgelegt. Bei 0°C gab man dazu 40 mg (0,3 mmol, 3 Mol%) wasserfreies Zinkchlorid und rührte 3 1/2 Stunden bei 0°C. Dann wurde innert einer Stunde auf -40°C abgekühlt, abfiltriert, mit wenig kaltem (bei etwa -10°C) Methanol gewaschen und schliesslich getrocknet. Dies ergab 2,5 g (75%ige Ausbeute) (all-E)-Vitamin A-Aldehyd-dimethylacetal mit Smp. 53-56°C; Gehalt nach HPLC: 98,4%.

Zur Analyse wurde eine Probe aus Methanol umkristallisiert. Diese Probe wies folgende physikalische bzw. analytische Daten auf:

Smp. 53-55°C; Gehalt nach HPLC: 99,7%; UV (n-Hexan): 324 nm (log ε = 4,70; $E_{1\,cm}^{1\%}$ = 1520);

Mikroanalyse:

| | | |
|---|---|---|
| Ber. | C 79,95% | H 10,37% |
| Gef. | C 79,66% | H 10,50% |

Beispiel 2

12'-Apo-β-carotinal-dimethylacetal

In einem mit mechanischem Rührer, Argonbegasung und Thermometer ausgestatteten 1,5 1-Vierhalssulfierkolben wurden 50 g (0,14 mol) 12'-Apo-β-carotinal und 31 ml (= 30,1 g, 0,28 mol) frisch destillierter Orthoameisensäure-trimethylester in 500 ml Methanol vorgelegt. Bei Raumtemperatur gab man nun zur resultierenden Suspension eine Lösung von 16 mg p-Toluolsulfonsäure-Monohydrat in 4 ml Methanol. Dabei lösten sich die roten Kristalle zum grössten Teil innert 20 Minuten auf; anschliessend begann sich ein oranger Niederschlag zu bilden. Nach 2 Stunden Rühren bei Raumtemperatur wurde auf ca. +5°C abgekühlt, 0,5 ml Triethylamin zugegeben, 15 Minuten bei 0°C nachgerührt, abgenutscht (Druckfilternutsche, unter Argon), mit wenig kaltem (-10°C) Methanol gewaschen und etwa 16 Stunden unter vermindertem Druck (Wasserstrahlvakuum) bei Raumtemperatur getrocknet. Dies ergab 52,3 g (90,5%ige Ausbeute) 12'-Apo-β-carotinal-dimethylacetal als oranges Pulver mit Smp. 77-78°C; Gehalt nach HPLC: 97,5% (sehr säurelabil);

UV (n-Hexan): 393 nm (log ε = 4,91; $E_{1\,cm}^{1\%}$ = 2045); 376 nm (log ε = 4,91; $E_{1\,cm}^{1\%}$ = 2045).
Mikroanalyse:

| | | |
|---|---|---|
| Ber. | C 81,77% | H 10,17% |
| Gef. | C 81,50% | H 9,84% |

Beispiel 3

8'-Apo-β-carotinal-dimethylacetal

In einem mit mechanischem Rührer, Argonbegasung und Thermometer ausgestatteten 350 ml-Vierhalssulfierkolben wurden 6,25 g (15 mmol) 8'-Apo-β-carotinal und 6,6 ml (= 6,4 g, 60 mmol, 4 Aeq.) Orthoameisensäure-trimethylester in 200 ml Methanol vorgelegt. Bei Raumtemperatur gab man eine Lösung von 20 mg p-Toluolsulfonsäure-Monohydrat in 10 ml Methanol dazu und liess 2 1/2 Stunden rühren. Dabei lösten sich die roten Kristalle langsam auf, und ein oranges Kristallisat begann sich zu bilden. Dann wurden 2 ml Triethylamin zugegeben, innert etwa 30 Minuten auf 0°C abgekühlt, abgenutscht, mit wenig kaltem (-10°C) Methanol gewaschen und kurz unter vermindertem Druck (Wasserstrahlvakuum) getrocknet. Nach 30 Minuten ergab dies 11,6 g methanolfeuchtes Acetal mit einem Gehalt nach HPLC von 94,8%. Zur Umkristallisation wurden die Kristalle in 200 ml Diethylether gelöst, und dann gab man innert 1 1/2 Stunden 600 ml Methanol zu, kühlte auf 0°C ab, filtrierte die Kristalle ab, und trocknete sie bei Raumtemperatur unter vermindertem Druck (Wasserstrahlvakuum) und kurz unter Hochvakuum. Dies ergab 5,9 g (84%ige Ausbeute) 8'-Apo-β-carotinal-dimethylacetal als rostrote Kristalle mit Smp. 131-132°C; Gehalt nach HPLC: 98,4%; UV (n-Hexan): 450 nm (log ε = 5,06; $E_{1\,cm}^{1\%}$ = 2476), 424 nm (log ε = 5,07; $E_{1\,cm}^{1\%}$ = 2543).
Mikroanalyse:

| | | |
|---|---|---|
| Ber. | C 83,06% | H 10,02% |
| Gef. | C 82,91% | H 10,13% |

Beispiel 4

4'-Apo-β-carotinal-dimethylacetal

In einem mit mechanischem Rührer, Argonbegasung und Thermometer ausgestatteten 500 ml-Vierhalssulfierkolben wurden 10 g (20,7 mmol) 4'-Apo-β-carotinal und 35 ml (0,31 mol, etwa 15 Aeq.) Orthoameisensäure-trimethylester in 250 ml Methanol vorgelegt. Zur resultierenden dunkelroten Suspension gab man bei Raumtemperatur eine Lösung von 25 mg p-Toluolsulfonsäure-Monohydrat in 15 ml Methanol zu, rührte 45 Minuten bei Raumtemperatur, dann während 1 1/2 Stunden bei 30-35°C; die dunkelrote Suspension wandelte sich in eine braune Suspension um. Anschliessend gab man 2 ml Triethylamin zu und kühlte auf 0°C ab. Das ausgefallene Rohprodukt wurde abgenutscht, mit wenig kaltem Methanol gewaschen und unter vermindertem Druck bei Raumtemperatur kurz getrocknet. Das so erhaltene methanolfeuchte Produkt (etwa 17,8 g; Gehalt nach HPLC: 91%) wurde in 600 ml Diethylether mit leichtem Erwärmen gelöst und bei Raumtemperatur innert 1 Stunde mit 1 Methanol (enthaltend 2%o Triethylamin) versetzt. Dann wurde abgenutscht und mit wenig kaltem (0°C) Methanol gewaschen. Dies ergab nach 2-stündiger Trocknung bei Raumtemperatur und unter vermindertem Druck 9,3 g (80,5%ige Ausbeute) 4'-Apo-β-carotinal-dimethylacetal als violette Kristalle mit einem Gehalt nach HPLC von 94,7%.

Für die analytischen Daten wurde eine Probe aus Diethylether (warm gelöst und auf 0°C abgekühlt) umkristallisiert: Gehalt nach HPLC: 95,7%; Smp. 186-188°C; UV (Dioxan): 500 nm (log ε = 4,93; $E_{1\,cm}^{1\%}$ = 1623), 468 nm (log ε = 4,99; $E_{1\,cm}^{1\%}$ = 1837), 283 nm (log ε = 4,37; $E_{1\,cm}^{1\%}$ = 444).

Beispiel 5

12,12'-Diapocarotinal-dimethylacetal ($C_{10}$-Dialdehyd-dimethylacetal)

In einem mit Magnetrührer und Argonbegasung ausgestatteten 500 ml-Rundkolben wurden 32,8 g (0,2 mol) $C_{10}$-Dialdehyd und 65 g Orthoameisensäure-trimethylester in 250 ml Methanol vorgelegt. Bei etwa 20°C gab man dazu 100 mg p-Toluolsulfonsäure-monohydrat, was eine leicht exotherme Reaktion auslöste. Das Reaktionsgemisch wurde mit einem kalten Wasserbad auf etwa 20-25°C gehalten. Die Suspension löste sich in etwa 5 Minuten auf. Dann wurde eine Stunde bei Raumtemperatur nachgerührt und anschliessend etwa 0,5 ml Triethylamin zugegeben. Nun wurde unter vermindertem Druck eingeengt und der anfallende Kristallbrei in 200 ml heissem n-Hexan gelöst, durch Watte heiss filtriert und stehen gelassen. Man liess die Lösung in einem Tiefkühlschrank etwa 16 Stunden bei -20°C stehen, filtrierte die resultierenden Kristalle ab, wusch sie mit n-Hexan bei -20°C und trocknete sie unter Wasserstrahlvakuum bis zur Gewichtskonstanz. Dies ergab 42,7 g (80%ige Ausbeute) $C_{10}$-Dialdehyd-dimethylacetal als hellgelbe, knackige Kristalle mit Smp. 68-69°C und einem Gehalt nach Gaschromatographie (GC) von etwa 96%); UV (Ethanol): 292 nm (log ε = 4,61; $E_{1\,cm}^{1\%}$ = 1602), 280 nm (log ε = 4,72; $E_{1\,cm}^{1\%}$ = 2046), 260 nm (log ε = 4,59; $E_{1\,cm}^{1\%}$ = 1508).
Mikroanalyse:

| | | |
|---|---|---|
| Ber. | C 65,60% | H 9,44% |
| Gef. | C 65,43% | H 9,14% |

Beispiel 6

8,8'-Diapocarotinal-dimethylacetal (Crocetindialdehyd-dimethylacetal)

In einem Magnetrührer und Argonbegasung ausgestatteten 500 ml-Rundkolben wurden 20,0 g (67,5 mmol) Crocetindialdehyd (Smp. 196-197°C) und 40 g (0,37 mol) Orthoameisensäure-trimethylester in 350 ml Methanol vorgelegt. Unter Rühren gab man bei Raumtemperatur 200 mg p-Toluolsulfonsäure-Monohydrat zu, rührte etwa 45 Minuten bei Raumtemperatur und etwa 1 1/2 Stunden bei 35-40°C, was eine gelb-orange Suspension ergab. Dann wurde auf 0°C abgekühlt, abfiltriert und mit kaltem Methanol (-10°C) gewaschen. Dies ergab 24,7 g (92%ige Ausbeute) Crocetindialdehyd-dimethylacetal als oranges Pulver, Smp. 136°C, mit einem Gehalt nach HPLC von 97,4%. Umkristallisation aus 150 ml heissem Ethylacetat und 150 ml Methanol unter Abkühlen bis auf -20°C ergab nach Filtration und Trocknung (Wasserstrahl-, gefolgt von Hochvakuum) 23,3 g (86%ige Ausbeute) Crocetindialdehyd-dimethylacetal als rostrote, knackige Kristalle, Smp. 138-139°C, mit einem Gehalt nach HPLC von 97,3%; UV (Ethanol): 422 nm (log ε = 5,12; $E_{1\,cm}^{1\%}$ = 3416), 397 nm (log ε = 5,11; $E_{1\,cm}^{1\%}$ = 3305), 377 nm (log ε = 4,89; $E_{1\,cm}^{1\%}$ = 1985), 232 nm (log ε = 4,20; $E_{1\,cm}^{1\%}$ = 405).
Mikroanalyse:

| | | |
|---|---|---|
| Ber. | C 74,19% | H 9,34% |
| Gef. | C 74,10% | H 9,47% |

B. Herstellung der Verbindungen der Formeln IV' und IV" aus den Polyen-(di-)O,O-dialkylacetalen der Formel II' bzw. II" und den 1-Alkoxy-1,3-dienen der Formel III

Beispiel 7

15-Methoxy-15,15'-dihydro-12'-apo-β-carotinal

In einem mit Magnetrührer und Thermometer ausgestatten Zweihalsrundkolben wurden unter Argon 2,38 g (7 mmol) Vitamin A-Aldehyd-dimethylacetal (HPLC: 97,8%) und 1,13 g (11,2 mmol) 1-Methoxy-2-methyl-1,3-butadien (GC: 98%) in 50 ml Toluol vorgelegt. Man kühlte das Gemisch auf -20°C ab, gab 15 mg (1 Mol%) p-Toluolsulfonsäure-Monohydrat zu und rührte das Gemisch 2 Stunden bei -20°C. Zwecks Feststellung des Ablaufs der Reaktion wurde dann eine Probe ausgenommen und mit 1 bis 2 Tropfen 90%iger wässriger Essigsäure hydrolysiert; das Gemisch wurde mit Wasser stark verdünnt, die wässrige Lösung mit wenig Toluol extrahiert und der Extrakt dünnschichtchromatographisch [DC; auf Silikagel ($SiO_2$)] untersucht, und zwar mit folgendem Ergebnis: DC ($SiO_2$): $R_f$ = etwa 0,3; Toluol/ Ethylacetat (19:1).
Zur Hydrolyse gab man zum Rundkolbeninhalt 10 ml Essigsäure/ Wasser (9:1) zu und rührte eine Stunde bei 0°C. Zur Aufarbeitung wurde auf 35 ml Wasser gegossen und zweimal mit je 35 ml, total 70 ml, Ethylacetat extrahiert. Dann wurden die vereinigten organischen Phasen dreimal mit je 35 ml, total 105 ml, Wasser, einmal mit gesättigter Natriumbicarbonat-lösung und einmal mit gesättigter Natriumchloridlösung gewaschen, über wasserfreiem Natriumsulfat getrocknet, filtriert und unter vermindertem Druck eingeengt. Dies ergab 3,0 g gelbes, öliges Rohprodukt, welches an 300 g Kieselgel (0,04-0,063 mm) mit Toluol/Ethylacetat (19:1 bis 9:1) chromatographiert wurde. Dies ergab 1,2 g (40%ige Ausbeute) (all-E)-15-Methoxy-15,15'-dihydro-12'-apo-β-carotinal mit einem Gehalt nach HPLC von 90,5%; UV (Cyclohexan, 2% Chloroform): 328 nm (log ε = 4,59; $E_{1\,cm}^{1\%}$ = 1007). Zur spektroskopischen Charakterisierung gelangte ein chromatographisch weitergereinigtes Material mit einem Gehalt nach HPLC von 94,8%: IR (Film): 1690 $cm^{-1}$ (CHO); Massenspektrum: 382 ($M^+$, 55), 299 (100).
Als Nebenprodukt wurden etwa 13% öliges (13Z)-15-Methoxy-15,15'-dihydro-12'-apo-β-carotinal isoliert. Zur Charakterisierung dieses Nebenproduktes gelangte eine Probe mit einem Gehalt nach HPLC von 95,4%:

$^1$H-NMR ($CDCl_3$, 250 MHz): 9,46 ppm (C$\underline{H}$O); IR (Film): 1689 $cm^{-1}$ (CHO);
Massenspektrum: 382 ($M^+$, 50), 299 (100).

Beispiel 8

12'-Methoxy-11',12'-dihvdro-8'-apo-β-carotinal

In einem mit Magnetrührer und Thermometer ausgestatteten 100 ml-Zweihalsrundkolben wurden 1,98 g (5 mmol) 12'-Apo-β-carotinal-dimethylacetal (HPLC: etwa 98%) und 0,78 g (8 mmol) 1-Methoxy-2-methyl-1,3-butadien (GC: 96%ig) in 40 ml n-Hexan vorgelegt. Bei -25°C gab man 34 mg (3,5 Mol%) p-Toluolsulfonsäure-Monohydrat zu und rührte 2 Stunden bei -25°C [DC ($SiO_2$) nach vorheriger Hydrolyse: Rf = etwa 0,3; Toluol/Ethylacetat (19:1)].

Zur Hydrolyse wurden dann zum Rundkolbeninhalt 10 ml Essigsäure/ Wasser (9:1) zugegeben, und das Gemisch wurde 30 Minuten bei 0°C gerührt. Zur Aufarbeitung wurde auf 25 ml Wasser gegossen und zweimal mit je 25 ml, total 50 ml, Ethylacetat extrahiert. Dann wurden die vereinigten organischen Phasen dreimal mit je 25 ml, total 75 ml, Wasser, einmal mit gesättigter Natriumbicarbonatlösung und einmal mit gesättigter Natriumchloridlösung gewaschen, über wasserfreiem Natriumsulfat getrocknet, filtriert und unter vermindertem Druck eingeengt. Dies ergab 2,9 g öliges, rotes Rohprodukt, welches an 70 g Kieselgel (0,04-0,063 mm) mit Toluol/Ethylacetat (19:1) chromatographiert wurde. Es resultierten 2,01 g (86%ige Ausbeute) 12'-Methoxy-11',12'-dihydro-8'-apo-β-carotinal als oranges Oel mit einem Gehalt nach HPLC von 96,2%. Die spektroskopischen Daten stammen aus einem anderen Ansatz (Gehalt nach HPLC: 96%):

UV (n-Hexan): 395 nm (log ε = 4,89; $E^{1\%}_{1\,cm}$ = 1752), 377 nm (log ε = 4,91; $E^{1\%}_{1\,cm}$ = 1794);
$^1$H-NMR (400 MHz, $CDCl_3$): 9,40 ppm (C$\underline{H}$O), 3,22 ppm (OC$\underline{H}_3$);
IR (Film): 1688 $cm^{-1}$ (CHO);
Massenspektrum: 448 ($M^+$, 75), 365 (100).

Beispiel 9

8'-Methoxy-7',8'-dihydro-4'-apo-β-carotinal

In einem mit Magnetrührer und Thermometer ausgestatteten 100 ml-Zweihalsrundkolben wurden unter Argon 2,36 g (5 mmol) 8'-Apo-β-carotinal-dimethylacetal (Gehalt nach HPLC: 98%) und 0,82 g (8 mmol, 1,6 Aeq.) 1-Methoxy-2-methyl-1,3-butadien (GC: 96%) in 50 ml Toluol vorgelegt. Bei -15°C wurden 30 mg (0,16 mmol, 3 Mol%) p-Toluolsulfonsäure-Monohydrat zugegeben, und das Gemisch wurde eine Stunde bei -15°C gerührt [DC ($SiO_2$) nach vorheriger Hydrolyse: $R_f$ = etwa 0,3; Toluol/Ethylacetat (19:1)].

Zur Hydrolyse gab man dann bei -15°C 10 ml Essigsäure/Wasser (9:1) zu, rührte 1 1/2 Stunden bei 0°C und arbeitete analog dem Beispiel 7 oder 8 auf. Dies ergab 3,2 g rohes 8'-Methoxy-7',8'-dihydro-4'-apo-β-carotinal [HPLC-Verhältnis Produkt: Nebenprodukt (Telomer) = 98,5:1,5] als roten, klebrigen Rückstand. Dieser wurde in 50 ml Methanol bei 45°C 45 Minuten digeriert, dann abgenutscht, mit kaltem (0°C) Methanol gewaschen und unter Hochvakuum getrocknet, was 1,96 g (73%ige Ausbeute) 8'-Methoxy-7',8'-dihydro-4'-apo-β-carotinal als orangerote Kristalle mit Smp. 152-153°C [HPLC-Verhältnis Produkt : Nebenprodukt = 95,8 : 1,4] ergab.

Zwecks Analyse wurde eine Probe (300 mg) aus heissem Ethanol umkristallisiert. Dies ergab 155 mg reines 8'-Methoxy-7',8'-dihydro-4'-apo-β-carotinal mit Smp. 158-159°C; Gehalt nach HPLC: 100%;

UV (Cyclohexan mit 3% Chloroform): 456 nm (log ε = 5,03; $E^{1\%}_{1\,cm}$ = 2105), 430 nm (log ε = 5,08; $E^{1\%}_{1\,cm}$ = 2335);
$^1$H-NMR (400 MHz, $CDCl_3$): 9,40 ppm (C$\underline{H}$O), 3,22 ppm (OC$\underline{H}_3$);
IR (KBr): 1680 $cm^{-1}$ (CHO);
Massenspektrum: 514 ($M^+$, 100), 431 (25);
Mikroanalyse:

| | | |
|---|---|---|
| Ber. | C 83,99% | H 9,79% |
| Gef. | C 83,69% | H 10,03% |

Beispiel 10

4'-Methoxy-β,ψ-carotin-16'-al

In einem 25 ml-Zweihalsrundkolben wurden unter Argon 2,32 g (4,25 mmol) 4'-Apo-β-carotinal-dimethylacetal (Gehalt nach HPLC: 97%) und 0,682 g (6,8 mmol, 1,6 Aeq.) 1-Methoxy-2-methyl-1,3-butadien (GC: 98%) in 40 ml Toluol vorgelegt. Bei -25°C gab man zum Gemisch 25 mg (0,13 mmol, 3 Mol%) p-Toluolsulfonsäure-Monohydrat, und

rührte das Gemisch 3 1/2 Stunden bei dieser Temperatur [DC ($SiO_2$) nach vorheriger Hydrolyse: $R_f$= etwa 0,3; Toluol/ Ethylacetat (19:1)].

Dann gab man zur Hydrolyse 20 ml Essigsäure/Wasser (9:1) zu und rührte eine Stunde bei 0°C. Eine extraktive Aufarbeitung wie in Beispiel 7 oder 8 beschrieben ergab 4,2 g Rohprodukt, welches an 400 g Kieselgel (0,04-0,063 mm) mit Toluol/Ethylacetat (19:1) chromatographiert wurde. Es resultierten 2,43 g roter Feststoff mit Smp. 148-154°C, welchen man in 25 ml Ethanol eine Stunde bei 50°C durch Rühren digerierte. Anschliessend wurde auf 0°C abgekühlt, filtriert und mit wenig Ethanol gewaschen. Nach Trocknen unter Hochvakuum erhielt man 1,36 g (54%ige Ausbeute) 4'-Methoxy-β,ψ-carotin-16'-al als rotbraune Kristalle mit Smp. 158-160°C; Gehalt nach HPLC: 97,1%.

Zur Analyse gelangte eine analytische Probe mit Smp. 161-163°C:

UV (Cyclohexan/3% Chloroform): 497 nm (log ε = 5,13; $E^{1\%}_{1\,cm}$ = 1986), 465 nm (log ε = 5,19; $E^{1\%}_{1\,cm}$ = 2258), 440 nm (log ε = 5,02; $E^{1\%}_{1\,cm}$ = 1550), 283 nm (log ε = 4,48; $E^{1\%}_{1\,cm}$ = 443);
$^1$H-NMR (250 MHz, $CDCl_3$): 9,40 ppm (C<u>H</u>O);
IR (KBr): 1688 $cm^{-1}$ (CHO);
Massenspektrum: 580 ($M^+$/4), 118 (100).
Mikroanalyse:

| | | |
|---|---|---|
| Ber. | C 84,77% | H 9,72% |
| Gef. | C 84,55% | H 9,42% |

Beispiel 11

11,12,11',12'-Tetrahydro-12,12'-dimethoxy-8,8'-diapocarotin-8,8'-dial

In einem mit Magnetrührer,Tropftrichter und Thermometer ausgestatteten 350 ml-Vierhalssulfierkolben wurden unter Argon 10,30 g (40 mmol) $C_{10}$-Dialdehyd-dimethylacetal (HPLC: 99,6%) in 200 ml Toluol vorgelegt. Man kühlte die Lösung auf -25°C ab und gab unter Rühren 76 mg (0,4 mmol, 1 Mol%) p-Toluolsulfonsäure zu, gefolgt von einer Lösung von 9,1 g (90 mmol, 2,25 Aeq.) 1-Methoxy-2-methyl-1,3-butadien in 16 ml Toluol. Die Zugabe dieser Lösung dauerte etwa 10 Minuten, währenddessen die Temperatur im Bereich von etwa -20°C bis -25°C gehalten wurde. Das Gemisch wurde eine weitere Stunde bei -20°C nachgerührt [DC ($SiO_2$) nach vorheriger Hydrolyse: $R_f$ = etwa 0,3 (Produkt) und etwa 0,5 (Acetal); Toluol/ Ethylacetat (4:1)].

Zur Hydrolyse wurden dann zum Kolbeninhalt 20 ml Essigsäure/ Wasser (9:1) zugegeben, und das Gemisch wurde 1 Stunde bei Raumtemperatur gerührt. Eine extraktive Aufarbeitung wie in Beispiel 7 oder 8 beschrieben ergab nach zweimaliger Chromatographie an 500 g Kieselgel (0,04-0,063 mm) mit Toluol/Ethylacetat (9:1) und einmaliger Chromatographie an 700 g Kieselgel (0,04-0,063 mm) mit Cyclohexan/Ethylacetat (3:1) 10,0 g (67%ige Ausbeute nach HPLC-Korrektur) gelbes, zum Teil festes 11,12,11',12'-Tetrahydro-12,12'-dimethoxy-8,8'-diapocarotin-8,8'-dial mit einem Gehalt an gewünschtem Endprodukt von 97,1% nach HPLC. Durch Kristallisation aus Ethanol konnte aus diesem Rohprodukt ein reineres Produkt, Smp. 105-107°C, erhalten werden, das zur Analyse gelangte:

UV (Cyclohexan/5% Chloroform): 300 mm (log ε = 4,57), 287 nm (log ε = 4,69);
IR (KBr): 1679 $cm^{-1}$ (CHO);
Massenspektrum: 360 ($M^+$, 1), 194 (100).

Beispiel 12

7,8,7',8'-Tetrahydro-8,8'-dimethoxy-4,4'-diapocarotin-4,4'-dial

In einem 200 ml-Vierhalskolben wurden unter Argon 4,04 g (10 mmol) Crocetindialdehyd-dimethylacetal (HPLC: 96,2%) in 50 ml Ethylacetat vorgelegt. Bei -15°C gab man unter Rühren 38 mg (2 Mol%) p-Toluolsulfonsäure, gefolgt von einer Lösung von 2,82 g (28 mmol, 2,8 Aeq.) 1-Methoxy-2-methyl-1,3-butadien in 4 ml Ethylacetat zu. Das Gemisch wurde 3 Stunden bei -15°C nachgerührt [DC ($SiO_2$) nach vorheriger Hydrolyse: Rf = etwa 0,3 (Produkt) und etwa 0,5 (Acetal); Toluol/Ethylacetat (9:1)].

Zur Hydrolyse wurden dann zum Kolbeninhalt 10 ml Essigsäure/ Wasser (9:1) zugegeben, und das Gemisch wurde 2 Stunden bei Raumtemperatur gerührt. Eine extraktive Aufarbeitung wie in Beispiel 7 oder 8 beschrieben ergab nach Chromatographie an 500 g Kieselgel (0,04-0,063 mm) mit Methylenchlorid/Ethylacetat (19:1) 2,4 g (43%ige Ausbeute nach HPLC-Korrektur) 7,8,7',8'-Tetrahydro-8,8'-dimethoxy-4,4'-diapocarotin-4,4'-dial als oranges Pulver (Gehalt an gewünschtem Endprodukt nach HPLC 89%).

Zur Analyse gelangte eine nochmals chromatographierte und aus Methylenchlorid/Ethanol umkristallisierte Probe mit Smp. 179-187°C (Isomerengemisch) und 91,7%iger Reinheit nach HPLC:

UV (Cyclohexan/2% Chloroform): 428 nm (log ε = 5,14), 402 nm (log ε = 5,12), 381 nm (log ε = 4,90);
IR (KBr): 1679 $cm^{-1}$ (CHO);
Massenspektrum: 492 ($M^+$, 100).

C. Herstellung der Polyen(di)aldehyde der Formeln I' und I" aus den Verbindungen der Formel IV' bzw. IV"

Beispiel 13

12'-Apo-β-carotinal

In einem 10 ml-Rundkolben wurden 1,20 g (2,82 mmol) (all-E)-15-Methoxy-15,15'-dihydro-12'-apo-β-carotinal (Gehalt nach HPLC: 90%) in 7 ml Ethanol vorgelegt und mit 0,11 ml (0,6 mmol, 20 Mol%) einer 5,4M Natriummethylatlösung in Methanol versetzt. Das Gemisch wurde eine Stunde bei Raumtemperatur gerührt [DC ($SiO_2$): $R_f$ = etwa 0,5; Toluol/Ethylacetat (19:1)].

Zur entstandenen tiefroten Suspension gab man zwecks Neutralisation 5 Tropfen Essigsäure, und dann kühlte man eine Stunde auf 0°C ab, filtrierte und wusch die resultierenden Kristalle mit wenig eiskaltem Methanol und Wasser und trocknete sie unter Hochvakuum bei Raumtemperatur bis zur Gewichtskonstanz. Dies ergab 0,76 g (75%ige Ausbeute) 12'-Apo-β-carotinal als tiefrote Kristalle mit Smp. 107-109°C; Gehalt nach HPLC: 98,1%.

UV (Cyclohexan, 2% Chloroform): 4,17 nm (log ε = 4,87; $E^{1\%}_{1\,cm}$ = 2114);
$^1$H-NMR (250 MHz, $CDCl_3$): 9,45 ppm (C<u>H</u>O);
IR (KBr): 1663 $cm^{-1}$ (CHO);
Massenspektrum: 350 ($M^+$, 100).
Mikroanalyse:

| | | |
|---|---|---|
| Ber. | C 85,66% | H 9,78% |
| Gef. | C 85,45% | H 9,49% |

Beispiel 14

8'-Apo-β-carotinal (Durchprozess aus 12'-Apo-β-carotinal-dimethylacetal und 1-Methoxy-2-methvl-1,3-butadien über 12'-Methoxy-11',12'-dihydro-8'-apo-β-carotinal

In einem mit Magnetrührer und Thermometer ausgestatteten 200 ml-Sulfierkolben wurden unter Argon 8,02 g (20 mmol) 12'-Apo-β-carotinaldimethylacetal (Gehalt nach HPLC: etwa 99%) und 2,96 g (29 mmol) 1-Methoxy-2-methyl-1,3-butadien (GC: etwa 96%) in 150 ml n-Hexan vorgelegt. Bei -25°C gab man zum Gemisch 40 mg (0,21 mmol, 1 Mol%) p-Toluolsulfonsäure-Monohydrat und rührte bei -22 bis -25°C 2 1/2 Stunden. (Das 12'-Apo-β-carotinal-dimethylacetal löst sich bei Raumtemperatur, fällt jedoch bei -25°C wieder aus). Bei der Reaktion löste sich die resultierende hellgelbe Suspension langsam auf unter Bildung einer dunkelgrünen Lösung, welche sich am Ende der Reaktion bräunlich färbte [DC ($SiO_2$) nach vorheriger Hydrolyse: Rf des Produktes = etwa 0,4; Rf des Ausgangsmaterials (Acetal) = etwa 0,5; Toluol/Ethylacetat (19:1)].

Zur Hydrolyse wurden dann bei -25°C 20 ml Essigsäure/Wasser (9:1) zugegeben und die Reaktionslösung eine Stunde bei 0°C gerührt.

Zur Aufarbeitung wurde auf 100 ml Wasser gegossen und mit je 100 ml, total 200 ml, n-Hexan extrahiert. Anschliessend wusch man die organische Phase der Reihe nach mit Wasser, mit 100 ml gesättigter Natriumbicarbonatlösung und mit 100 ml gesättigter Natriumchloridlösung. Dann wurde die organische Phase über wasserfreiem Natriumsulfat getrocknet, filtriert und unter vermindertem Druck eingeengt. Zum resultierenden Rückstand wurden etwa 80 ml Ethanol gegeben, und man engte zur Entfernung von Resten Hexan die Lösung nochmals ein. Dies ergab 10,6 g rohes, noch ethanolfeuchtes 12'-Methoxy-11',12'-dihydro-8'-apo-β-carotinal als rot-oranges, dickflüssiges Oel [HPLC: 86,4% Produkt, 5% Nebenprodukt (Telomer); Verhältnis Produkt: Nebenprodukt = 94,5:5,5]. Dieses Oel wurde nun in 170 ml Ethanol gelöst und in einen mit Magnetrührer ausgestatteten 250 ml-Rundkolben unter Argon übertragen. Unter Rühren gab man bei Raumtemperatur zur rotorangen Lösung 0,4 ml (etwa 2 mmol, 10 Mol%) einer 5,4M-Lösung von Natriummethylat in Methanol zu. Dabei verfärbte sich die Reaktionslösung sofort tiefdunkelrot, und nach etwa 5 bis 10 Minuten fing eine Auskristallisation an. Man rührte etwa eine Stunde bei Raumtemperatur [DC ($SiO_2$): Rf (Zwi-

schenprodukt) = etwa 0,4, $R_f$ (Produkt) = etwa 0,7; Toluol/Ethylacetat (19:1)].

Nun wurde mit einem Eisbad bei 0°C abgekühlt (etwa 1/2 Stunde) und mit 0,5 g (etwa 8 mmol) Essigsäure in 2-3 ml Ethanol neutralisiert. Nun wurden 3,5 ml Wasser zugetropft und bei +5°C (Kühlschrank) etwa 16 Stunden stehengelassen. Danach wurde der Niederschlag mit einer Glasfilternutsche abfiltriert und zweimal mit je 20 ml, total 40 ml, Ethanol/Wasser (19:1) bei 0°C, dann mit 70 ml Wasser bei Raumtemperatur und noch zweimal mit je 10 ml, total 20 ml, Ethanol/Wasser (19:1) bei 0°C gewaschen. Nach Trocknung unter Wasserstrahlvakuum bei 45°C und unter Hochvakuum bei Raumtemperatur wurden 7,53 g (83%ige Ausbeute) 8'-Apo-β-carotinal als blaues, kristallines Pulver mit Smp. 137-138°C und einem Gehalt nach HPLC von 92% erhalten.

Zur weiteren Reinigung wurden 7,48 g obigen Pulvers in 110 ml Aceton etwa 10 Minuten unter Rückfluss gelöst. Zur Lösung gab man dann durch den Kühler unter Rückfluss und starkem Rühren 8 ml Wasser tropfenweise zu, was eine Kristalliation auslöste, und dann wurde langsam auf 0°C abgekühlt. Nachdem im Eisbad etwa 2 Stunden gerührt worden war, filtrierte man vom Niederschlag ab und wusch ihn zweimal mit je 30 ml, total 60 ml, Ethanol/Wasser (9:1) bei 0°C, zweimal mit je 30 ml, total 60 ml, Wasser und schliesslich dreimal mit je 5 ml, total 15 ml, Ethanol/Wasser (9:1) bei 0°C. Nach Trocknung unter Wasserstrahlvakuum bei 45°C und unter Hochvakuum bei Raumtemperatur erhielt man 6,79 g (81%ige Ausbeute) reines 8'-Apo-β-carotinal als violette Kristalle mit Smp. 141-142°C und einem Gehalt nach HPLC von 99,5%.

Die analytischen Daten entstammen einem analogen Ansatz: Smp. 141-143°C; Gehalt nach HPLC: 95,4%; UV (Cyclohexan mit 3% Chloroform):

460 nm (log ε = 5,03, $E^{1\%}_{1\,cm}$ = 2562); $^1$H-NMR (400 MHz, $CDCl_3$): 9,45 ppm (C<u>H</u>O);
IR (KBr): 1669, 1609 $cm^{-1}$;
Massenspektrum: 416 ($M^+$, 100);
Mikroanalyse:

| | | |
|---|---|---|
| Ber. | C 86,48% | H 9,68% |
| Gef. | C 86,23% | H 9,54% |

Beispiel 15

4'-Apo-β-carotinal

In einem mit Magnetrührer ausgestatteten 100 ml-Rundkolben wurden unter Argon 1,96 g (3,65 mmol) 8'-Methoxy-7',8'-dihydro-4'-apocarotinal (Gehalt nach HPLC: 95,7%) in 50 ml Ethylacetat/Methanol (1:1) vorgelegt. Zum Gemisch gab man 0,1 ml (0,5 mmol, etwa 15 Mol%) einer 5,4M-Lösung von Natriummethylat in Methanol und rührte eine Stunde bei 50°C [DC ($SiO_2$): $R_f$ (Ausgangsmaterial) = etwa 0,4; $R_f$ (Produkt) = 0,5; Toluol/Ethylacetat (9:1)].

Nun wurde mit 0,1 ml Essigsäure neutralisiert, 2 Stunden bei 0°C gerührt, mit wenig Methanol/Wasser (9:1) abgenutscht und mit eiskaltem Methanol gewaschen. Nach Trocknung unter Wasserstrahlvakuum und anschliessend unter Hochvakuum bei Raumtemperatur erhielt man 1,62 g (90%ige Ausbeute) 4'-Apo-β-carotinal als dunkle Kristalle mit Smp. 161-162°C; Gehalt nach HPLC: 97,7%; UV (Cyclohexan mit 3% Chloroform): 520 nm (Schulter, log ε = 5,01; $E^{1\%}_{1\,cm}$ = 2100), 492 nm (log ε = 5,12; $E^{1\%}_{1\,cm}$ = 2709), 296 nm (log ε = 4,44; $E^{1\%}_{1\,cm}$ = 566);

$^1$H-NMR (400 MHz, $CDCl_3$): 9,35 ppm (C<u>H</u>O);
IR (KBr): 1670, 1611 $cm^{-1}$;
Massenspektrum: 482 ($M^+$, 30), 119 (100);
Mikroanalyse:

| | | |
|---|---|---|
| Ber. | C 87,08% | H 9,60% |
| Gef. | C 86,60% | H 9,56% |

Beispiel 16

4'-Apo-β-carotinal (Durchprozess aus 8'-Apo-β-carotinal-dimethylacetal und 1-Methoxy-2-methyl-1,3-butadien über 8'-Methoxy-7'.8'-dihydro-4'-apo-β-carotinal)

In einem mit Magnetrührer ausgestatteten 250 ml-Zweihalsrundkolben wurden unter Argon 6,4 g (13,6 mmol) 8'-Apo-β-carotinal-dimethylacetal (Gehalt nach HPLC: etwa 98%) und 2,22 g (21,7 mmol, 1,6 Aeq.) 1-Methoxy-2-methyl-1,3-butadien (GC: etwa 96%) vorgelegt. Bei -15°C gab man zum Gemisch 80 mg (0,4 mmol, 3 Mol%) p-Toluolsulfonsäure-Monohydrat und rührte 1 1/2 Stunden bei dieser Temperatur [DC ($SiO_2$) nach vorheriger Hydrolyse: $R_f$ (Acetal) = etwa 0,4; $R_f$ (Produkt) = etwa 0,3; Toluol/Ethylacetat (19:1)]. Dann wurden zwecks Hydrolyse 20 ml Essigsäure/Wasser (9:1) bei -15°C zugegeben, und man rührte das Gemisch 2 Stunden bei 0°C. Anschliessend wurde auf Wasser gegossen und zweimal mit je 100 ml, total 200 ml, Ethylacetat extrahiert und eingeengt. Dies ergab 8,7 g rohes 8'-Methoxy-7',8'-dihydro-4'-apo-β-carotinal als rotes Harz mit einem Gehalt nach HPLC von 91,6%.

Dieser Rückstand wurde nun ohne Reinigung in 160 ml Methanol/ Ethylacetat (3:1) aufgenommen, wobei nur ein Teil des Rückstands löslich war, und die Lösung mit Rückstand wurde mit 1,4 ml (7,5 mmol, etwa 0,4 Aeq.) einer 5,4M-Lösung von Natriummethylat in Methanol versetzt und 5 Stunden bei 50°C gerührt [DC ($SiO_2$): Rf (8'-Methoxy-7',8'-dihydro-4'-apo-β-carotinal) = etwa 0,3; $R_f$ (Produkt) = etwa 0,5-0,6; Toluol/Ethylacetat (19:1)].

Nun wurde mit 1,5 ml Essigsäure neutralisiert, auf 0°C abgekühlt, abgenutscht und das Filtergut mit kaltem (0°C) Methanol/ Wasser (9:1) und kaltem Methanol gewaschen und unter Wasserstrahlvakuum und unter Hochvakuum bei Raumtemperatur bis zur Gewichtskonstanz getrocknet. Dies ergab 4,78 g (70%ige Ausbeute bezogen auf das 8'-Apo-β-carotinaldimethylacetal) 4'-Apo-β-carotinal als dunkelviolettes Pulver mit Smp. 142-145°C und einem Gehalt nach HPLC von 96%.
Die weiteren analytischen Angaben entsprachen denjenigen, die am Ende des Beispiels 15 vorhanden sind.

Beispiel 17

3',4'-Didehydro-β,ψ-carotin-16'-al

In einem 25 ml-Rundkolben wurde unter Argon 1,00 g (1,64 mmol) 4'-Methoxy-β,ψ-carotin-16'-al (Gehalt nach HPLC: 95,4%) in 14 ml Toluol/ Ethanol (1:1) vorgelegt. Zum Gemisch gab man 0,1 ml (0,5 mmol, 30 Mol%) einer 5,4M-Natriummethylat-Lösung in Methanol, und man rührte das Gemisch 3 Stunden bei 35°C [DC ($SiO_2$): $R_f$ = etwa 0,6; Toluol/Ethylacetat (19:1)].

Zur Neutralisation gab man 0,1 ml Essigsäure zu, kühlte auf 0°C ab, filtrierte und wusch die resultierenden Kristalle mit kaltem Methanol und Wasser. Dies ergab nach Trocknen unter Hochvakuum bei Raumtemperatur 880 mg (97%ige Ausbeute) 3',4'-Didehydro-β,ψ-carotin-16'-al (Torularhodinaldehyd) als dunkelrote Kristalle mit Smp. 176-177°C und einem Gehalt nach HPLC von 99,2%.

UV (Cyclohexan/3% Chloroform): 514 nm (log ε = 5,19; $E^{1\%}_{1\,cm}$ = 2820), 326 nm (log ε = 4,55; $E^{1\%}_{1\,cm}$ = 644);
$^1$H-NMR (250 MHz, $CDCl_3$): 9,45 ppm (C<u>H</u>O);
IR (KBr): 1660 $cm^{-1}$ (CHO);
Massenspektrum: 548 ($M^+$, 100).
Mikroanalyse:

| | | |
|---|---|---|
| Ber. | C 87,53% | H 9,55% |
| Gef. | C 87,41% | H 9,35% |

Beispiel 18

Crocetindialdehyd

In einem 250 ml-Rundkolben wurden unter Argon 9,6 g (25,8 mmol) 11,12,11',12'-Tetrahydro-12,12'-dimethoxy-8,8'-diapocarotin-8,8'-dial (Gehalt nach HPLC: 97,1%) in 100 ml Methanol vorgelegt. Zum Gemisch gab man bei Raumtemperatur 0,75 ml (3,9 mmol, 15 Mol%) einer 5,4M-Natriummethylat-Lösung in Methanol, und man rührte das Gemisch 1 Stunde bei Raumtemperatur und 2 Stunden bei 35°C [DC ($SiO_2$): Rf = etwa 0,6 (Produkt) und etwa 0,5 (Ausgangsmaterial); Toluol/Ethylacetat (19:1)].

Zur Neutralisation gab man 5 ml Essigsäure zu, kühlte auf 0°C ab, filtrierte und wusch die resultierenden roten Kristalle der Reihe nach mit 40 ml Methanol/Wasser (9:1) bei 0°C, 100 ml Wasser und nochmals 40 ml Methanol/ Wasser (9:1) bei 0°C. Dies ergab nach Trocknen unter Wasserstrahlvakuum bei 35°C und Hochvakuum bei Raumtemperatur 6,99 g (91%ige Ausbeute) Crocetindialdehyd als dunkelrote, glänzende Kristalle mit Smp. 195-196°C und einem Gehalt nach HPLC von 99,7%.

UV (Cyclohexan/5% Chloroform): 464 nm (log ε = 5,14), 436 nm (log ε = 5,13), 413 nm (log ε = 4,91);
IR (KBr): 1668 $cm^{-1}$ (CHO);
Massenspektrum: 296 ($M^+$, 100);

Mikroanalyse:

| | | |
|---|---|---|
| Ber. | C 81,04% | H 8,16% |
| Gef. | C 80,92% | H 8,19% |

Beispiel 19

Crocetindialdehyd (Durchprozess aus $C_{10}$-Dialdehyd-dimethylacetal und 1-Methoxv-2-methyl-1,3-butadien über 11,12,11',12'-Tetrahvdro-12.12'-dimethoxy-8,8'-diapocarotin-8.8'-dial

In einem mit Magnetrührer, Tropftrichter und Thermometer ausgestatteten 500 ml-Vierhalssulfierkolben wurden unter Argon 25,74 g (100 mmol) $C_{10}$-Dialdehyd-dimethylacetal (Gehalt nach HPLC: 99,5%) in 250 ml Toluol vorgelegt. Bei -25°C wurden dann 190 mg (1 Mol%) p-Toluolsulfonsäure-Monohydrat, gefolgt von einer Lösung von 23,65 g (235 mmol, 2,35 Aeq.) 1-Methoxy-2-methyl-1,3-butadien in 40 ml Toluol zugegeben, und zwar innert 20 Minuten bei -25°C. Man rührte das Gemisch bei -25°C weitere 45 Minuten [DC ($SiO_2$) nach vorheriger Hydrolyse: $R_f$ = etwa 0,5 (Produkt) und etwa 0,3 (Acetal); Toluol/Ethylacetat (4:1)].

Zur Hydrolyse wurden dann bei -25°C 75 ml Essigsäure/Wasser (9:1) zugegeben, und die Reaktionslösung wurde 2 Stunden bei Raumtemperatur gerührt. Die anschliessende Aufarbeitung erfolgte analog dem Beispiel 7 oder 8, was 42 g rohes 11,12,11',12'-Tetrahydro-12,12'-dimethoxy-8,8'-diapocarotin-8,8'-dial als gelbes Oel ergab. Dieses wurde in 250 ml Methanol aufgenommen, und die Lösung unter Rühren mit 2,75 ml (15 Mol%) einer 5,4M-Natriummethylat-Lösung in Methanol versetzt. Man rührte das Gemisch 1 1/2 Stunden bei 35°C [DC ($SiO_2$): $R_f$ = etwa 0,6 (Produkt) und etwa 0,4 (Zwischenprodukt); Toluol/Ethylacetat (4:1)].

Zu der nun angefallenen roten Suspension gab man 10 ml Essigsäure. Man kühlte das Gemisch auf -10°C ab, rührte es 2 Stunden bei dieser Temperatur und filtrierte die resultierenden roten Kristalle ab. Anschliessend wurden die Kristalle der Reihe nach mit 50 ml Methanol/Wasser (9:1) bei 0°C, 100 ml Wasser bei Raumtemperatur und 50 ml Methanol/Wasser (9:1) bei 0°C gewaschen und unter Wasserstrahlvakuum bei 35°C und Hochvakuum bei Raumtemperatur bis zur Gewichtskonstanz getrocknet. Auf diese Weise erhielt man 21,8 g (73%ige Ausbeute) Crocetindialdehyd als dunkelrote, glänzende Kristalle mit Smp. 193-194°C und einem Gehalt nach HPLC von 99,3%.

Beispiel 20

4,4'-Diapocarotinal

In einem 25 ml-Rundkolben wurden unter Argon 350 mg (0,75 mmol) 7,8,7',8'-Tetrahydro-8,8'-dimethoxy-4,4'-diapocarotin-4,4'-dial (Gehalt nach HPLC: 91,7%) in 18 ml Ethanol vorgelegt. Zur Lösung gab man 0,28 ml (1,5 mmol, 2 Aeq.) einer 5,4M-Natriummethoxid-Lösung in Methanol, und man rührte das Gemisch 3 Stunden bei 50°C. Es resultierte eine dunkle Suspension [DC ($SiO_2$): Rf = etwa 0,5 (Produkt) und etwa 0,3 (Ausgangsmaterial); Toluol/Ethylacetat (9:1)].

Nach Abkühlen der Suspension auf 0°C, Abfiltrieren der Kristalle, Waschen der Reihe nach mit je 5 ml Methanol, Wasser und Methanol und Trocknen unter Hochvakuum bei Raumtemperatur erhielt man 295 mg (88%ige Ausbeute) 4,4'-Diapocarotinal als dunkelblaue Kristalle mit Smp. 226°C und einem Gehalt nach HPLC von 96,3%.

UV (Cyclohexan/2% Chloroform): 537 nm (log ε = 5,14; $E^{1\%}_{1\,cm}$ = 3190), 501 nm (log ε = 5,20; $E^{1\%}_{1\,cm}$ = 3700), 471 nm (log ε = 5,04; $E^{1\%}_{1\,cm}$ = 2565);
IR (KBr): 1680 $cm^{-1}$ (CHO);
Massenspektrum: 428 ($M^+$, 100).

## Patentansprüche

**1.** Verfahren zur Herstellung eines Polyenaldehyds oder -dialdehyds der allgemeinen Formel

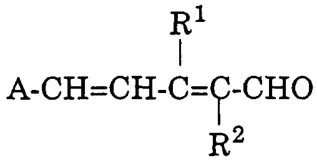

I'

oder

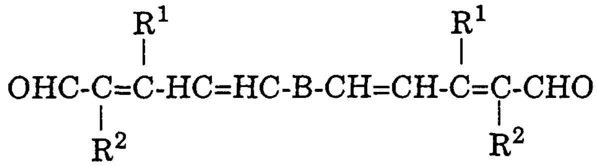

I"

worin

| | |
|---|---|
| A | eine monovalente, gegebenenfalls methylsubstituierte, konjugierte Polyengruppe, |
| B | eine bivalente, gegebenenfalls methylsubstituierte, konjugierte Polyengruppe und |
| $R^1$ und $R^2$ | jeweils Wasserstoff oder Methyl bedeuten, |

wobei sich die Gruppe(n) $-CH=CH-C(R^1)=C(R^2)-CHO$ jeweils in der(den) Endstellung(en) der konjugierten Kette der Gruppe A bzw. B befindet(n),

**dadurch gekennzeichnet**, dass man ein Polyen-O,O-dialkylacetal oder -di(O,O-dialkylacetal) der allgemeinen Formel

$$A\text{-}CH(OR^3)_2 \qquad \text{II'}$$

bzw.

$$(R^3O)_2HC\text{-}B\text{-}CH(OR^3)_2 \qquad \text{II''}$$

worin

| | |
|---|---|
| A und B | die oben angegebenen Bedeutungen besitzen, wobei sich in diesem Fall die Gruppe(n) $-CH(OR^3)_2$ jeweils in der(den) Endstellung(en) der konjugierten Kette der Gruppe A bzw. B befindet(n) und |
| $R^3$ | $C_{1-6}$-Alkyl bedeutet, |

mit einem 1-Alkoxy-1,3-dien der allgemeinen Formel

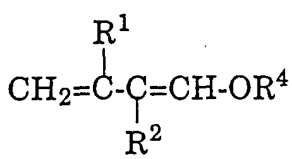

III

worin

| | |
|---|---|
| $R^1$ und $R^2$ | die oben angegebenen Bedeutungen besitzen und |
| $R^4$ | $C_{1-6}$-Alkyl bedeutet, |

in Gegenwart einer Brönstedsäure umsetzt, das Reaktionsgemisch hydrolysiert, und von der so hergestellten Verbindung der allgemeinen Formel

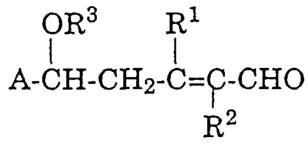

IV'

bzw.

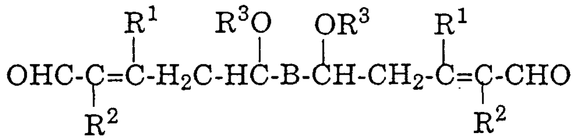

IV''

worin A, B, $R^1$, $R^2$ und $R^3$ die oben angegebenen Bedeutungen besitzen, wobei sich in diesem Fall die Gruppe (n) $-CH(OR^3)-CH_2-C(R^1)=C(R^2)-CHO$ jeweils in der(den) Endstellung(en) der konjugierten Kette der Gruppe A bzw. B befindet (n), unter basischen in Gegenwart eines Alkalimetallalkoholats als Base den Alkohol $R^3OH$ abspaltet.

2. Verfahren nach Anspruch 1, **dadurch gekennzeichnet**, dass als Polyen-O,O-dialkylacetal oder -di(O,O-dialkylacetal) eine Verbindung der allgemeinen Formel

$$R - CH(OR^3)_2 \qquad II$$

worin

R eine Gruppe (a), (b) oder (c)

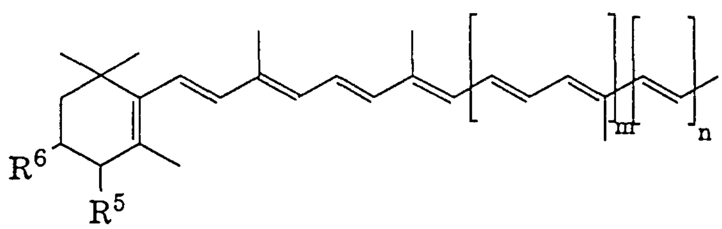

(a)

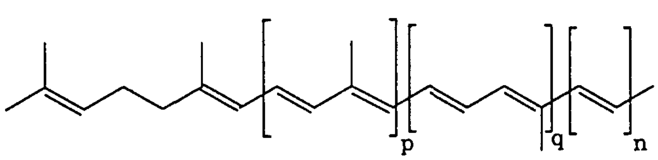

(b)

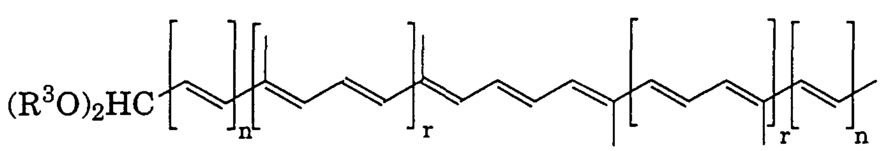

(c)

bedeutet, in der

$R^3$ $C_{1-6}$-Alkyl,

$R^5$ und $R^6$ unabhängig voneinander Wasserstoff, eine gegebenenfalls geschützte Hydroxygruppe oder eine gegebenenfalls geschützte Oxogruppe,

m 0, 1, 2, 3 oder 4,

n 0 oder 1,

p 0, 1 oder 2,

q 0, 1, 2 oder 3 und

r 0, 1 oder 2 bedeuten,

nach Durchführung des in Anspruch 1 definierten mehrstufigen Verfahrens in das entsprechende Produkt der allgemeinen Formel

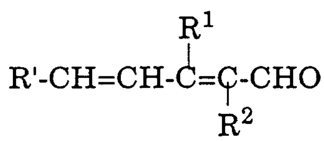

I

worin R' eine Gruppe (a) oder (b) bzw. eine Gruppe (c')

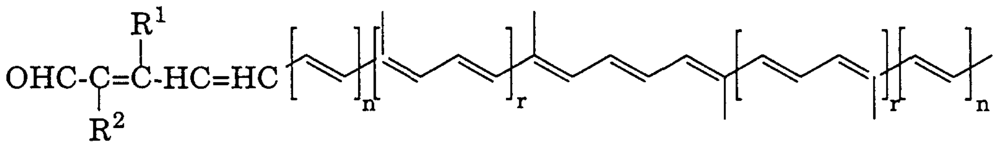

(c')

bedeutet,
übergeführt wird, und dass man im Falle des Vorhandenseins einer Gruppe (a) im Produkt der Formel I gewünschtenfalls eine allfällig vorhandene Schutzgruppe abspaltet.

**3.** Verfahren nach Anspruch 2, **dadurch gekennzeichnet**,dass R eine Gruppe (a) bedeutet, in der $R^5$ und $R^6$ beide Wasserstoff und n O bedeuten, oder R eine Gruppe (c) bedeutet, in der beide n O bedeuten, und in beiden Fällen $R^1$ und $R^2$ Wasserstoff resp. Methyl bedeuten.

**4.** Verfahren nach einem der Ansprüche 1 bis 3, **dadurch gekennzeichnet**, dass als Brönstedsäure p-Toluolsulfonsäure, Methansulfonsäure, Trifluormethansulfonsäure, Schwefelsäure oder Trifluoressigsäure verwendet wird.

**5.** Verfahren nach einem der Ansprüche 1 bis 4, **dadurch gekennzeichnet**, dass die Brönstedsäure in einer katalytischen Menge eingesetzt wird, welche zwischen-etwa 0,5 und 5 Molprozent bezogen auf die eingesetzte Menge des Polyen-O,O-dialkylacetals oder -di(O,O-dialkylacetals) der Formel II', II" bzw. II beträgt.

**6.** Verfahren nach einem der Ansprüche 1 bis 5, **dadurch gekennzeichnet**, dass man 1,05 bis 1,6 Aequivalente 1-Alkoxy-1,3-dien pro Aequivalent Polyen-O,O-dialkylacetal bzw. 2,1 bis 3,2 Aequivalente 1-Alkoxy-1,3-dien pro Aequivalent Polyen-di(O,O-dialkylacetal) umsetzt.

**7.** Verfahren nach einem der Ansprüche 1 bis 6, **dadurch gekennzeichnet**, dass man das Polyen-O,O-dialkylacetal oder -di(O,O-dialkylacetal) der Formel II', II" bzw. II mit dem 1-Alkoxy-1,3-dien der Formel III in einem organischen Lösungsmittel bei Temperaturen im Bereich von -60°C bis +60°C, wobei als organisches Lösungsmittel n-Pentan, n-Hexan, Cyclohexan, Methylenchlorid, Chloroform, Diethylether, tert.Butylmethylether, Tetrahydrofuran, Acetonitril oder Toluol verwendet wird.

**8.** Verfahren nach Anspruch 7, **dadurch gekennzeichnet**, dass die Umsetzung im Temperaturbereich von etwa -20°C bis zur Raumtemperatur erfolgt.

**9.** Verfahren nach einem der Ansprüche 1 bis 8, **dadurch gekennzeichnet**, dass man unmittelbar nach Beendigung der Reaktion des Polyen-O,O-dialkylacetals oder -di(O,O-dialkylacetals) der Formel II', II" bzw. II mit dem 1-Alkoxy-1,3-dien der Formel III das daraus resultierende Zwischenprodukt im Reaktionsgemisch selber hydrolysiert, indem man zum Reaktionsgemisch eine wässrige Lösung einer schwachen Säure, vorzugsweise leicht verdünnte wässrige Essigsäure, gibt und das Gemisch anschliessend im Temperaturbereich von 0°C bis zur Raumtemperatur rührt.

**10.** Verfahren nach einem der Ansprüche 1 bis 9, **dadurch gekennzeichnet**, dass man die Abspaltung des Alkohols $R^3OH$ von der Verbindung der Formel IV' oder IV" durchführt, indem man die in einem organischen Lösungsmittel gelöste Verbindung der Formel IV' oder IV" in Gegenwart einer katalytischen Menge des Alkalimetallalkoholats, vorzugsweise Natriummethylat, Natriumethylat, Kaliummethylat, Kaliumethylat oder Kalium-tert.butylat, als Base umsetzt.

**11.** Verbindungen der allgemeinen Formeln

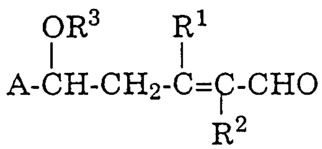

IV'

und

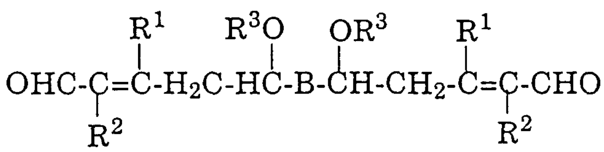

IV"

worin

A eine Gruppe (a) oder (b)

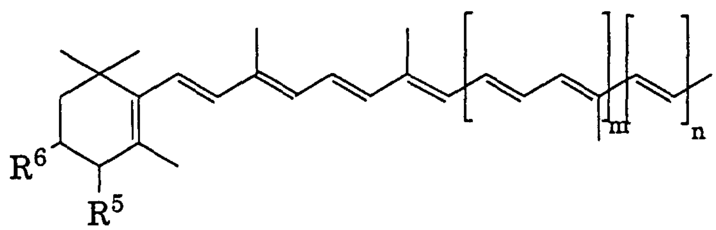

(a)

oder

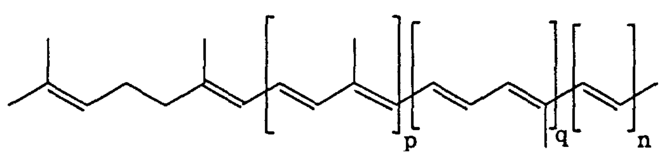

(b)

B eine Gruppe (c")

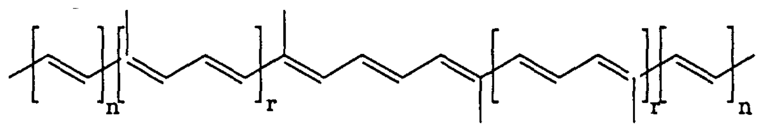

(c")

| | |
|---|---|
| $R^1$ und $R^2$ | jeweils Wasserstoff oder Methyl, |
| $R^3$ | $C_{1-6}$-Alkyl, |
| $R^5$ und $R^6$ | unabhängig voneinander Wasserstoff, eine gegebenenfalls geschützte Hydroxygruppe oder eine gegebenenfalls geschützte Oxogruppe, |
| m | 0,1,2,3 oder 4, |
| n | 0 oder 1, |
| p | 0, 1 oder 2, |
| q | 0, 1, 2 oder 3 und |
| r | 0 oder 1 bedeuten, |

mit der Massgabe, dass in der Gruppe (a) m und n nicht gleichzeitig O sind.

**12.** Eine Verbindung gemäss Anspruch 11, ausgewählt aus

12'-Methoxy-11',12'-dihydro-8'-apo-β-carotinal,
8'-Methoxy-7',8'-dihydro-4'-apo-β-carotinal,
4'-Methoxy-β,ψ-carotin-16'-al,
11,12,11',12'-Tetrahydro-12,12'-dimethoxy-8,8'-diapocarotin-8,8'-dial sowie
7,8,7',8'-Tetrahydro-8,8'-dimethoxy-4,4'-diapocarotin-4,4'-dial.

**Claims**

**1.** A process for the manufacture of a polyene aldehyde or polyene dialdehyde of the general formula

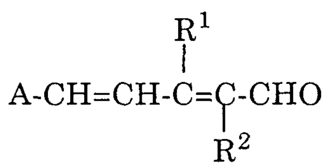

I'

or

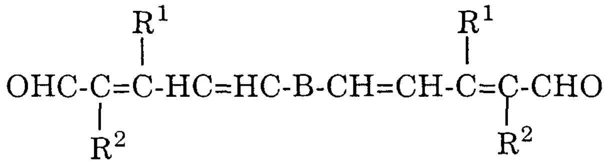

I"

wherein

| | |
|---|---|
| A | signifies a monovalent, optionally methyl-substituted, conjugated polyene group, |
| B | signifies a bivalent, optionally methyl-substituted, conjugated polyene group and |
| $R^1$ and $R^2$ | each signify hydrogen or methyl, with the -CH=CH-C($R^1$)=C($R^2$)-CHO group(s) in each case being situated in the terminal position(s) of the conjugated chain of group A or B, |

which process comprises reacting a polyene O,O-dialkyl acetal or di(O,O-dialkyl acetal) of the general formula

$$A\text{-}CH(OR^3)_2 \qquad II'$$

or

$$(R^3O)_2HC\text{-}B\text{-}CH(OR^3)_2 \qquad II''$$

wherein

A and B have the significances given above, with in this case the $-CH(OR^3)_2$ group(s) being situated in the terminal position(s) of the conjugated chain of group A or B and

$R^4$ signifies $C_{1\text{-}6}$-alkyl,

with a 1-alkoxy-1,3-diene of the general formula

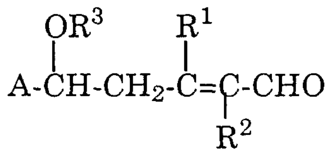

IV'

or

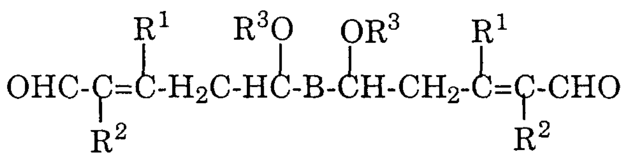

IV''

wherein

$R^1$ and $R^2$ have the significances given above and

$R^4$ signifies $C_{1\text{-}6}$-alkyl,

in the presence of a Brönsted acid, hydrolyzing the reaction mixture and cleaving off the alcohol $R^3OH$ under basic conditions, in the presence of an alkali metal alcoholate as the base, from the thus-produced compound of the general formula

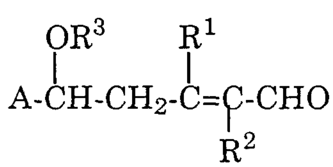

IV'

or

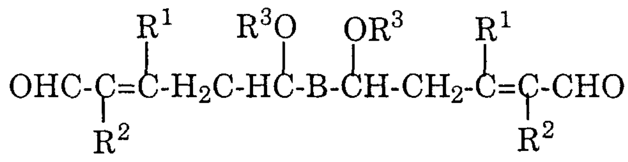

IV"

wherein
A, B, $R^1$, $R^2$ and $R^3$ have the significances given above, with in this case the $-CH(OR^3)-CH_2-C(R^1)=C(R^2)-CHO$ group(s) being situated in the terminal position(s) of the conjugated chain of group A or B.

**2.** A process according to claim 1, wherein the polyene O,O-dialkyl acetal or di(O,O-dialkyl acetal) is a compound of the general formula

$$R - CH(OR^3)_2 \qquad II$$

wherein

R signifies a group (a), (b) or (c)

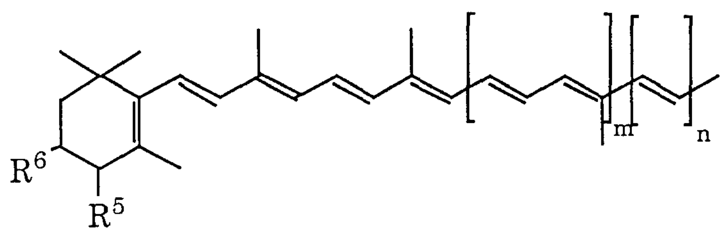

(a)

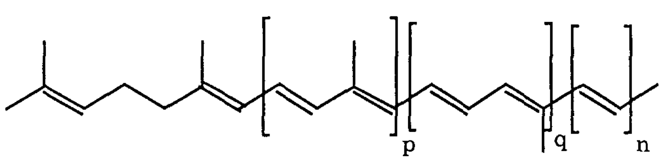

(b)

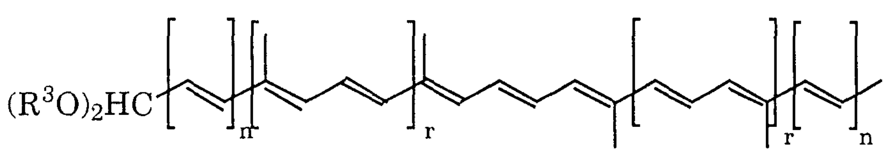

(c)

in which

- $R^3$ signifies $C_{1-4}$-alkyl,
- $R^5$ and $R^6$ each independently signify hydrogen, an optionally protected hydroxy group or an optionally protected oxo group,
- m signifies 0, 1, 2, 3 or 4,
- n signifies 0 or 1,
- p signifies 0, 1 or 2,

q signifies 0, 1, 2 or 3 and
r signifies 0, 1 or 2,

and is converted after carrying out the multistage process defined in claim 1 into the corresponding product of the general formula

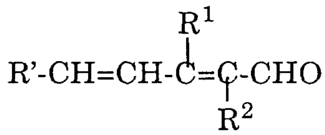

I

wherein R' signifies a group (a) or (b) or a group (c')

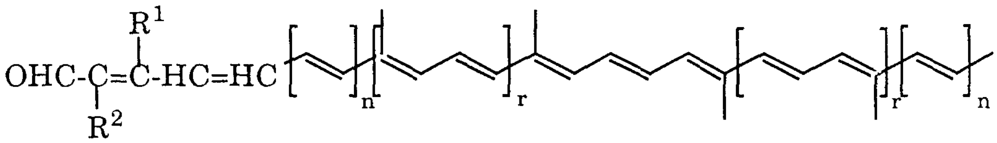

(c')

and, when a group (a) is present in the product of formula I, a protecting group present is cleaved off, if desired.

**3.** A process according to claim 2, wherein R signifies a group (a) in which $R^5$ and $R^6$ both signify hydrogen and n signifies 0 or R signifies a group (c) in which both n's signify 0 and in both cases $R^1$ and $R^2$ signify hydrogen and methyl, respectively.

**4.** A process according to any one of claims 1 to 3, wherein p-toluenesulphonic acid, methanesulphonic acid, trifluoromethanesulphonic acid, sulphuric acid or trifluoroacetic acid is used as the Brönsted acid.

**5.** A process according to any one of claims 1 to 4, wherein the Brönsted acid is used in a catalytic amount which is between 0.5 and 5 mol% based on the amount of polyene O,O-dialkyl acetal or di(O,O-dialkyl acetal) of formula II', II" or II, as appropriate, which is used.

**6.** A process according to any one of claims 1 to 5, wherein 1.05 to 1.6 equivalents of 1-alkoxy-1,3-diene are reacted per equivalent of polyene O,O-dialkyl acetal or 2.1 to 3.2 equivalents of 1-alkoxy-1,3-diene are reacted per equivalent of polyene di(O,O-dialkyl acetal).

**7.** A process according to any one of claims 1 to 6, wherein the polyene O,O-dialkyl acetal or di(O,O-dialkyl acetal) of formula II', II" or II, as appropriate, is reacted with the 1-alkoxy-1,3-diene of formula III in an organic solvent at temperatures in the range of -60°C to +60°C, with n-pentane, n-hexane, cyclohexane, methylene chloride, chloroform, diethyl ether, tert.butyl methyl ether, tetrahydrofuran, acetonitrile or toluene being used as the organic solvent.

**8.** A process according to claim 7, wherein the reaction is effected in the temperature range of -20°C to room temperature.

**9.** A process according to any one of claims 1 to 8, wherein immediately after completion of the reaction of the polyene O,O-dialkyl acetal or di(O,O-dialkyl acetal) of formula II', II" or II, as appropriate, with the 1-alkoxy-1,3-diene of formula III the intermediate resulting therefrom is itself hydrolyzed in the reaction mixture by adding to the reaction mixture an aqueous solution of a weak acid, preferably slightly diluted aqueous acetic acid, and subsequently stirring the mixture in the temperature range of 0°C to room temperature.

**10.** A process according to any one of claims 1 to 9, wherein the cleavage of the alcohol $R^3OH$ from the compound

of formula IV' or IV" is carried out by reacting the compound of formula IV' or IV" dissolved in an organic solvent in the presence of a catalytic amount of the alkali metal alcoholate, preferably sodium methylate, sodium ethylate, potassium methylate, potassium ethylate or potassium tert.butylate, as the base.

**11.** Compounds of the general formulae

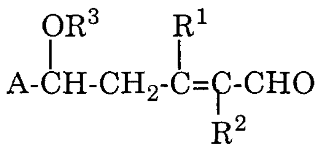

IV'

and

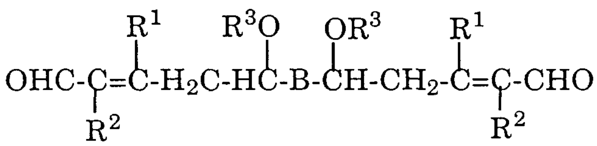

IV"

wherein

A signifies a group (a) or (b)

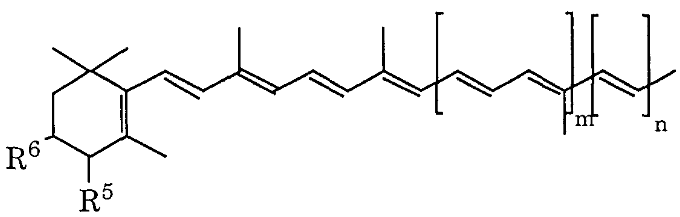

(a)

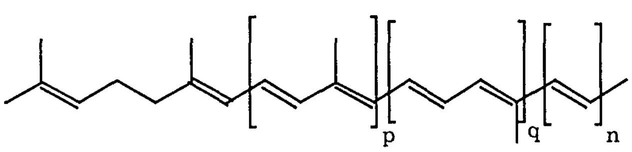

(b)

B signifies a group (c")

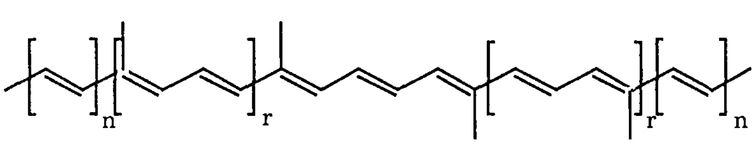

(c")

| | |
|---|---|
| $R^1$ and $R^2$ | each signify hydrogen or methyl, |
| $R^3$ | signifies $C_{1-6}$-alkyl, |
| $R^5$ and $R^6$ | each independently signify hydrogen, an optionally protected hydroxy group or an optionally protected oxo group, |
| m | signifies 0, 1, 2, 3 or 4, |
| n | signifies 0 or 1, |
| P | signifies 0, 1 or 2, |
| q | signifies 0, 1, 2 or 3 and |
| r | signifies 0 or 1, |

with the proviso that in the group (a) m and n are not simultaneously 0.

**12.** A compound according to claim 11, selected from

12'-methoxy-11',12'-dihydro-8'-apo-β-carotenal,
8'-methoxy-7',8'-dihydro-4'-apo-β-carotenal,
4'-methoxy-β,ψ-caroten-16'-al,
11,12,11',12'-tetrahydro-12,12'-dimethoxy-8,8'-diapocarotene-8,8'-dial and
7,8,7',8'-tetrahydro-8,8'-dimethoxy-4,4'-diapocarotene-4,4'-dial.

## Revendications

**1.** Procédé pour la préparation d'un aldéhyde ou dialdéhyde polyénique de formule générale

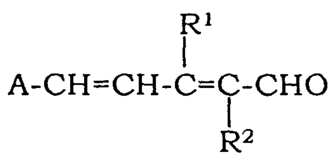

I'

ou

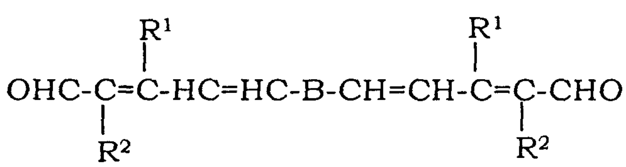

I"

formules dans lesquelles

| | |
|---|---|
| A | représente un groupe polyénique conjugué monovalent, éventuellement substitué par le groupe méthyle, |
| B | représente un groupe polyénique conjugué divalent, éventuellement substitué par le groupe méthyle et |
| $R^1$ et $R^2$ | représentent chacun un atome d'hydrogène ou le groupe méthyle, |

le(s) groupe(s) $-CH=CH-C(R^1)=C(R^2)-CHO$ se trouvant chaque fois dans la(les) position(s) terminale(s) de la chaîne conjuguée du groupe A ou B,
**caractérisé en ce que** l'on fait réagir un O,O-dialkylacétal ou di(O,O-dialkylacétal) polyénique de formule générale

$$A\text{-}CH(OR^3)_2 \qquad II'$$

ou

$$(R^3O)_2HC\text{-}B\text{-}CH(OR^3)_2 \qquad II''$$

formules dans lesquelles

A et B 3 ont les significations données plus haut, en ce cas le(s) groupe(s) $-CH(OR^3)_2$ se trouvant chaque fois dans la(les) position(s) terminale(s) de la chaîne conjuguée du groupe A ou B, et

$R^3$ représente un groupe alkyle en $C_1$-$C_6$,

avec un 1-alcoxy-1,3-diène de formule générale

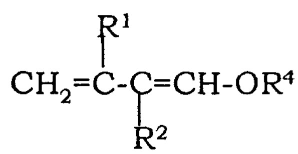

III

dans laquelle

$R^1$ et $R^2$ ont les significations données plus haut et

$R^4$ représente un groupe alkyle en $C_1$-$C_6$,

en présence d'un acide de Brönsted, on soumet à une hydrolyse le mélange réactionnel et on élimine l'alcool $R^3OH$ du composé ainsi obtenu, de formule générale

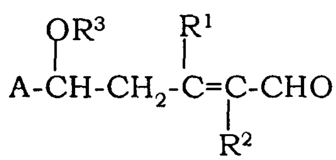

IV'

ou

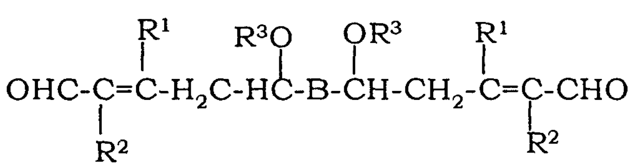

IV"

formules dans lesquelles A, B, $R^1$, $R^2$ et $R^3$ ont les significations données plus haut, en ce cas le(s) groupe(s) $-CH(OR^3)-CH_2-C(R^1)=C(R^2)-CHO$ se trouvant chaque fois dans la(les) position(s) terminale(s) de la chaîne conjuguée du groupe A ou B, dans des conditions basiques, en présence d'un alcoolate de métal alcalin en tant que base.

**2.** Procédé selon la revendication 1, **caractérisé en ce que** l'on convertit en tant que O,O-dialkylacétal ou di(O,O-dialkylacétal) polyénique un composé de formule générale

$$R\text{-}CH(OR^3)_2 \qquad II$$

dans laquelle

R représente un groupe (a), (b) ou (c)

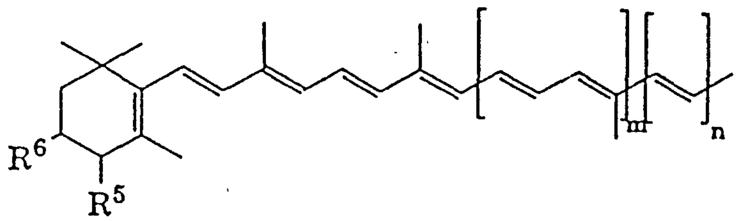

(a)

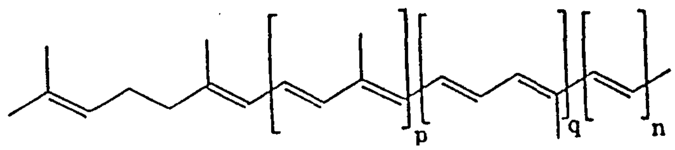

(b)

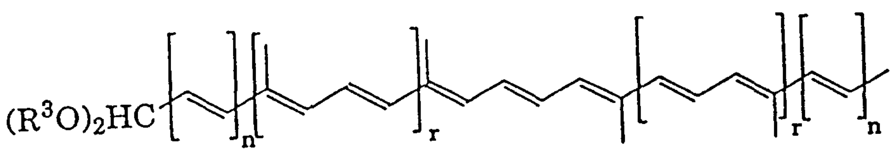

(c)

formules dans lesquelles

$R^3$ représente un groupe alkyle en $C_1$-$C_6$,

$R^5$ et $R^6$ représentent, indépendamment l'un de l'autre, un atome d'hydrogène, un groupe hydroxy éventuellement protégé ou un groupe oxo éventuellement protégé,

m représente 0, 1, 2, 3 ou 4

n représente 0 ou 1,

P représente 0, 1 ou 2,

q représente 0, 1, 2 ou 3 et

r représente 0, 1 ou 2,

après avoir exécuté le processus en plusieurs étapes défini dans la revendication 1, en le produit correspondant de formule générale

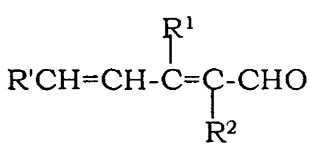

I

dans laquelle R' représente un groupe (a) ou (b) ou bien un groupe (c')

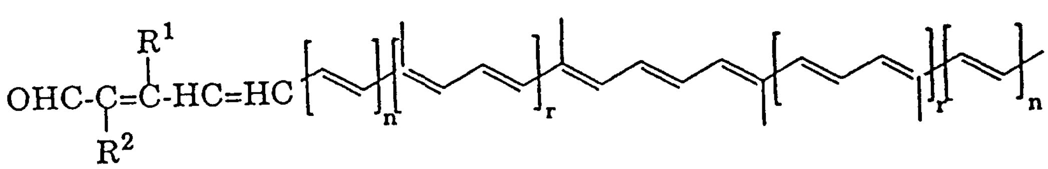

(c')

et en ce que, dans le cas de la présence d'un groupe (a) dans le produit de formule I, on élimine si on le désire un groupe protecteur éventuellement présent.

3. Procédé selon la revendication 2, **caractérisé en ce que** R représente un groupe (a) dans lequel $R^5$ et $R^6$ représentent l'un et l'autre un atome d'hydrogène et n est égal à 0, ou R représente un groupe (c) dans lequel les deux indices n représentent chacun 0, et dans les deux cas $R^1$ et $R^2$ représentent un atome d'hydrogène ou le groupe méthyle.

4. Procédé selon l'une des revendications 1 à 3, **caractérisé en ce que** l'on utilise comme acide de Brônsted l'acide p-toluènesulfonique, l'acide méthanesulfonique, l'acide trifluorométhanesulfonique, l'acide sulfurique ou l'acide trifluoroacétique.

5. Procédé selon l'une des revendications 1 à 4, **caractérisé en ce que** l'acide de Brônsted est utilisé en une quantité catalytique, qui représente entre 0,5 et 5 % en moles par rapport à la quantité utilisée du O,O-dialkylacétal ou di (O,O-dialkylacétal) polyénique de formule II', II" ou II.

6. Procédé selon l'une des revendications 1 à 5, **caractérisé en ce que** l'on fait réagir 1,05 à 1,6 équivalent de 1-alcoxy-1,3-diène par équivalent de O,O-dialkylacétal polyénique ou 2,1 à 3,2 équivalents de 1-alcoxy-1,3-diène par équivalent de di(O,O-dialkylacétal) polyénique.

7. Procédé selon l'une des revendications 1 à 6, **caractérisé en ce que** l'on met en réaction le O,O-dialkylacétal ou di(O,O-dialkylacétal) polyénique de formule II', II" ou II avec le 1-alcoxy-1,3-diène de formule III dans un solvant organique, à des températures dans la plage de -60°C à +60°C, en utilisant comme solvant organique le n-pentane, le n-hexane, le cyclohexane, le chlorure de méthylène, le chloroforme, l'oxyde d'éthyle, l'éther tert-butylméthylique, le tétrahydrofuranne, l'acétonitrile ou le toluène.

8. Procédé selon la revendication 7, **caractérisé en ce que** l'on effectue la réaction dans la plage de températures allant de -20°C à la température ambiante.

9. Procédé selon l'une des revendications 1 à 8, **caractérisé en ce que**, immédiatement après la fin de la réaction du O,O-dialkylacétal ou di(O,O-dialkylacétal) polyénique de formule II', II" ou II avec le 1-alcoxy-1,3-diène de formule III, on soumet le produit intermédiaire résultant à une hydrolyse dans le mélange réactionnel lui-même, en ajoutant au mélange réactionnel une solution aqueuse d'un acide faible, de préférence de l'acide acétique aqueux légèrement dilué, et en agitant ensuite le mélange dans la plage de températures allant de 0°C à la température ambiante.

10. Procédé selon l'une des revendications 1 à 9, **caractérisé en ce que** l'on effectue l'élimination de l'alcool $R^3H$ du composé de formule IV' ou IV" en faisant réagir le composé de formule IV' ou IV", dissous dans un solvant organique, en présence d'une quantité catalytique de l'alcoolate de métal alcalin, de préférence le méthylate de sodium, l'éthylate de sodium, le méthylate de potassium, l'éthylate de potassium ou le tert-butylate de potassium, en tant que base.

11. Composés de formules générales

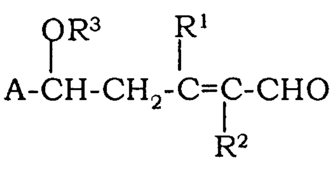

IV'

et

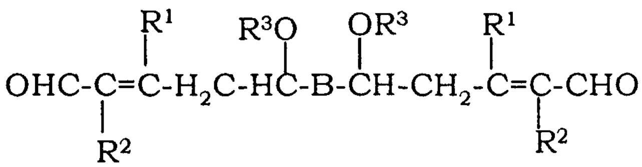 IV''

dans lesquelles

A représente un groupe (a) ou (b)

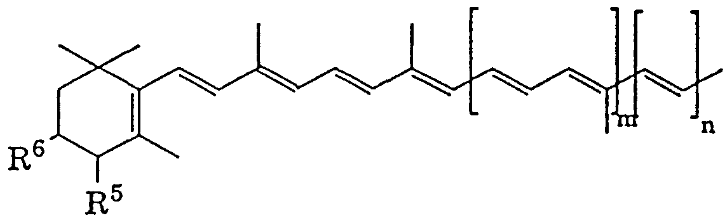 (a)

ou

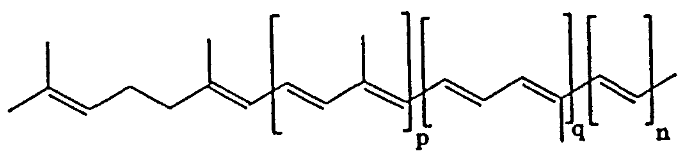 (b)

B représente un groupe (c")

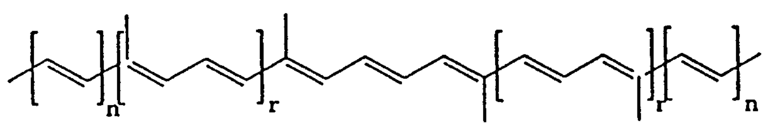

(c")

- $R^1$ et $R^2$ représentent chacun un atome d'hydrogène ou le groupe méthyle,
- $R^3$ représente un groupe alkyle en $C_1$-$C_6$,
- $R^5$ et $R^6$ représentent, indépendamment l'un de l'autre, un atome d'hydrogène, un groupe hydroxy éventuellement protégé ou un groupe oxo éventuellement protégé,
- m représente 0, 1, 2, 3 ou 4,
- n représente 0 ou 1,
- p représente 0, 1 ou 2,
- q représente 0, 1, 2 ou 3, et
- r représente 0 ou 1,

étant entendu que dans le groupe (a) m et n ne sont pas simultanément zéro.

**12.** Composé selon la revendication 11, choisi parmi

le 12'-méthoxy-11',12'-dihydro-8'-apo-β-caroténal,
le 8'-méthoxy-7',8'-dihydro-4'-apo-β-caroténal,
le 4'-méthoxy-β,ψ-carotén-16'-al,

le 11,12,11',12'-tétrahydro-12,12'-diméthoxy-8,8'-diapocarotène-8,8'-dial ainsi que le 7,8,7',8'-tétrahydro-8,8'-diméthoxy-4,4'-diapocarotène-4,4-dial.